# EUROPEAN PATENT APPLICATION

(11) **EP 4 596 050 A1**
(43) Date of publication of application: **06.08.2025**
(21) Application number: 24155610.9
(22) Date of filing: 02.02.2024
(51) Int. Cl.: A61P 35/00, C12Q 1/6806

(54) **METHODS OF PREPARING PROCESSED RNA SAMPLES AND THEIR USE IN PREPARING RNA VACCINES**

(71) Applicant: WOBBLE GENOMICS LIMITED, Edinburgh, Lothian EH4 2HS (GB)
(72) Inventor: KUO, Richard Izen, Edinburgh, EH4 2HS (GB)
(74) Representative: Boult Wade Tennant LLP

(57) **Abstract**

Methods for determining a set of RNA sequences associated with a disease and producing a database of RNA sequences associated with a disease are provided. Methods for discovering disease biomarkers and diagnosing disease are also provided. The present invention also provides methods for processing RNA and cDNA sequences and uses of these methods. Methods for producing RNA vaccines are also provided.

## Description

### FIELD OF THE INVENTION

The invention relates to methods for analysing nucleic acid samples including methods for identifying and using biomarkers. The invention also relates to methods for determining a set of RNA sequences associated with a disease or condition and producing a database of RNA sequences associated with a disease or condition. Methods for diagnosing a disease and producing an RNA vaccine for a subject with a disease are also provided.

### BACKGROUND

RNA sequencing has become a powerful tool for understanding biology (Stark, R., Grzelak, M. & Hadfield, J. RNA sequencing: the teenage years. Nat. Rev. Genet. 20, 631-656 (2019)). Its applications range from drug development to improving agriculture. Most cells and tissues share many of the same highly expressed genes which are commonly known as house-keeping genes. These genes are typically responsible for basic cell functions and thus do not provide cell specific characteristics. Since these house-keeping genes typically make up a large fraction of RNA within a sample, RNA sequencing data is usually dominated by sequencing reads from these non-informative RNA. This phenomenon results in two main negative effects on generating good results from RNA sequencing projects; first, genes and isoforms which are specific to the condition in question are difficult to detect, and second, the data generated is, in large part, redundant.

The first main negative effect has two consequences. The first is that the amount of sequencing required to detect genes of interest must be large enough to handle sampling inefficiencies caused by the low relative abundance of genes of interest. The second being that, in some cases, low abundance target genes may be simply impractical to identify. This can be evidenced by the still ongoing efforts to annotate the human genome where even after thousands of sequencing projects the full human transcriptome is still elusive with novel isoforms and genes being reported with regularity. Since eukaryotic transcriptomes derive their complexity from alternative splicing which generates combinatorial permutations, the search for novel RNA will likely be a constant endeavour.

These two consequences ultimately hamper scientific progress by limiting the abilities of researchers to produce ideal results from their sequencing experiments. These consequences also contribute to the impracticality of applying RNA sequencing toward a wider range of uses. For instance, for use in diagnostics and treatment tracking where the volume of sequencing required would be both time and cost prohibitive.

The second main negative effect (generation of redundant data) also has two main consequences. The first is that more data requires more processing time which increases overall cost and time of RNA sequencing experiments. These costs are both in terms of energy from additional computation required and work time from bioinformaticians that are tasked with processing the data. The second consequence is that redundant data results in the need for more storage. As sequencing is becoming more widespread, data storage has become a significant problem. For RNA sequencing technology to take on more roles, more efficient data generation is necessary to reduce storage requirements.

Accordingly, it is with these problems in mind that the present invention has been devised.

### SUMMARY OF THE INVENTION

The invention is based on methods that take advantage of their ability to generate full length sequences from RNA extracted from blood without fragmenting RNA or cDNA products before sequencing. This provides a transcriptome representing any RNA that make its way into the circulatory system. These include RNA from typical blood cells like red blood cells, white blood cells, other immune cells, etc. These would also include any other cells that are typically uncommon in the circulatory system such as cancer cells or cells that somehow dislodged into the circulatory system. This also includes extracellular RNA which could have originated from any cell within the body. By detecting RNA from all these sources and at full length the invention enables each RNA to be ascribed to a cell/tissue of origin as well as a state of cell or tissue behaviour. The transcription start site, end site, and splicing are features which typically represent unique combinations used by different cell types. This information can also be used to directly identify the protein isoform that would be translated from messenger RNA. The ability to detect protein isoforms and identify those that are associated with a disease allow for the development of treatments such as RNA vaccines.

It should be borne in mind that the various aspects have been devised so as to be advantageously combined and all such combinations are envisaged within the scope of the invention. It should also be appreciated that options described in relation to one area of improvement will apply *mutatis mutandis* to other areas; e.g. sample types, nucleic acid types etc. as appropriate.

The invention provides a method for analysing a nucleic acid sample, the method comprising:
(i) processing the nucleic acid sample; and
(ii) sequencing the processed nucleic acid.

The nucleic acid sample may be obtained from a biological sample of any suitable form including any material, biological fluid, tissue, or cell obtained or otherwise derived from a subject. The nucleic acid sample may be obtained from (cancer) cells or genetic material (DNA or RNA) derived from the (cancer) cells, to include cell-free genetic material (e.g. found in the peripheral blood). The nucleic acid sample may be obtained from a biopsy sample, optionally a solid biopsy sample and/or a liquid biopsy sample. The nucleic acid sample may be obtained from biological fluid or a fluid or lysate generated from a biological material. The nucleic acid sample may be obtained from blood. The nucleic acid sample may be obtained by extracting RNA from a biological sample (e.g. blood) obtained from a subject. cDNA may then be synthesized using the RNA as a template (i.e. by reverse transcription).

Blood samples may be readily and frequently obtained, allowing for repeated and non-invasive sampling of a patient.

The invention provides a method for analysing a nucleic acid sample, the method comprising:
(i) providing an RNA sample extracted from a blood sample obtained from a subject;
(ii) processing the RNA sample, optionally comprising synthesizing cDNA using the RNA as a template; and
(iii) sequencing the processed RNA or cDNA.

The blood sample may be whole blood.

Sequencing the processed RNA or cDNA generates a sequencing output. The sequencing output may provide a transcriptome representing any RNA in the blood. Thus, the sequencing output from the methods defined herein may comprise sequences of RNA from blood cells, other cells dislodged into the blood, and/or cancer cells, as well as extracellular RNA. The sequencing output may be used to ascribe each RNA sequence/molecule to a cell or tissue of origin and/or to a state of cell or tissue behaviour.

The invention provides a method for analysing a nucleic acid sample, the method comprising:
(i) providing a nucleic acid sample comprising an RNA molecule, optionally
   wherein the nucleic acid sample is extracted from a blood sample obtained from a subject;
(ii) processing the nucleic acid sample comprising the RNA molecule, optionally comprising synthesizing cDNA using the RNA molecule as a template; and
(iii) sequencing the processed RNA or cDNA, wherein the sequencing output is used to identify the cell type and/or tissue type from which the RNA molecule is derived.

The invention provides a method for analysing a nucleic acid sample, the method comprising:
(i) providing a nucleic acid sample comprising an RNA molecule, optionally
   wherein the nucleic acid sample is extracted from a blood sample obtained from a subject;
(ii) processing the nucleic acid sample comprising the RNA molecule, optionally comprising synthesizing cDNA using the RNA molecule as a template; and
(iii) sequencing the processed RNA or cDNA, wherein the sequencing output is used to identify the protein isoform encoded by the RNA molecule.

The sequencing output from the methods defined herein may be used to identify the transcription start site, end site and/or splicing. The methods defined herein may comprise identifying at least one transcription start site, end site and/or splice junction after sequencing the processed RNA or cDNA (i.e. in the sequencing output), optionally identifying the transcription start site, end site and all splice junctions in one or more (2, 3, 4, 5, 10, 20, 100 or 1000 or more) transcript(s). These features typically represent a unique combination used by different cell types enabling the cell type to be identified. This information can also be used to identify the protein isoform that would be translated from an RNA sequence/molecule/transcript. Thus, the sequencing output may be used to identify the presence of an RNA transcript sequence. The sequencing output may be used to identify one or more isoforms of a protein. The protein may have 2, 3, 4, 5, 10, 15 or 20 or more isoforms and the method may identify which of the protein isoforms is encoded by the RNA molecule.

The RNA (transcript) sequence/molecule or set of RNA sequences may be associated with a disease or condition. An RNA (transcript) sequence/molecule or set of RNA sequences associated with a disease or condition is an RNA (transcript) sequence/molecule or set of RNA sequences that is correlated with the disease or condition. An RNA (transcript) sequence/molecule or set of RNA sequences associated with a disease or condition may be (uniquely) present in a subject with the disease or condition, optionally absent in a subject without the disease or condition. An RNA (transcript) sequence/molecule or set of RNA sequences associated with a disease or condition may be (uniquely) absent in a subject with the disease or condition, optionally present in a subject without the disease or condition. The RNA (transcript) sequence may have a coding sequence that is disease (for example cancer) specific. An RNA (transcript) sequence may encode a protein isoform that is associated with the disease or condition. One or more peptide sequence(s) may be identified in the protein isoform that are present only in cells from subjects with the disease, optionally cancer cells. One or more peptide sequence(s) may be identified in the protein isoform that are antigenic peptides. These peptide sequences (antigenic peptides) are encoded by sections of the RNA (transcript) sequence.

The invention provides a method for determining a set of RNA sequences associated with a disease or condition, the method comprising:
(a) providing a (test) RNA sample obtained from a subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript.

The RNA sample may be extracted from any material, biological fluid, tissue, or cell obtained or otherwise derived from the subject. The sample may be a (solid) biopsy sample. The sample may be a liquid biopsy sample. The sample may be obtained from a biological fluid or a fluid or lysate generated from a biological material. The RNA sample may be extracted from a blood sample obtained from the subject.

The invention provides a method for determining a set of RNA sequences associated with a disease or condition, the method comprising:
(a) providing a (test) RNA sample extracted from a blood sample obtained from a subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript.

The subject may have the disease or condition or the subject may be pregnant and the foetus may have the disease or condition. Each RNA sequence in the set may encode an antigenic peptide. Each RNA sequence in the set may be (uniquely) expressed in a subject with the disease or condition and, optionally, is not expressed in subjects without the disease or condition. By "set of RNA sequences" is meant 2 or more, optionally 3, 4, 5, 6, 7, 8, 9, 10, 20, 50 or 100 or more RNA sequences. Each RNA sequence in the set may be more than 10bp, 20 bp, 50 bp, 100bp, 500bp, 1000 bp long, optionally 10 to 10000 bp, 20 to 1000bp or 50 to 500 bp long. Each RNA sequence in the set may be less than 10 bp, 20 bp, 50 bp, 100 bp, 500 bp, or 1000 bp long. One or more, optionally 2, 3, 4, 5, 6, 7, 8, 9, 10, 20, 50 or 100 or more RNA sequences from the set may be used to produce a (first) RNA vaccine for the subject.

The invention provides a method for producing an RNA vaccine for a subject with a disease, the method comprising:
(a) providing a (test) RNA sample obtained from the subject;
(b) processing the RNA sample, optionally comprising synthesizing cDNA using the RNA as a template;
(c) sequencing the processed RNA or cDNA, wherein the sequencing output is used to identify the presence of an RNA (transcript) sequence associated with the disease; and
(d) using the identified RNA (transcript) sequence to produce a first RNA vaccine for the subject.

The RNA sample may be extracted from any material, biological fluid, tissue, or cell obtained or otherwise derived from the subject. The sample may be a (solid) biopsy sample. The sample may be a liquid biopsy sample. The sample may be obtained from a biological fluid or a fluid or lysate generated from a biological material. The RNA sample may be extracted from a blood sample obtained from the subject.

The invention provides a method for producing an RNA vaccine for a subject with a disease, the method comprising:
(a) providing a test RNA sample extracted from a blood sample obtained from the subject;
(b) processing the RNA sample, optionally comprising synthesizing cDNA using the RNA as a template;
(c) sequencing the processed RNA or cDNA, wherein the sequencing output is used to identify the presence of an RNA (transcript) sequence associated with the disease; and
(d) using the identified RNA (transcript) sequence to produce a first RNA vaccine for the subject.

The invention provides a method for producing an RNA vaccine for a subject with a disease, the method comprising:
(a) providing a (test) RNA sample extracted from a blood sample obtained from the subject;
(b) processing the RNA sample, optionally comprising synthesizing cDNA using the RNA as a template;
(c) sequencing the processed RNA or cDNA, wherein the sequencing output is used to identify the presence of an RNA (transcript) sequence associated with the disease; and
(d) using the identified RNA (transcript) sequence to produce a first RNA vaccine for the subject;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

The use of certain sample types such as blood samples is advantageous for continuous monitoring of subjects. Therefore, the method may further comprise:
(e) providing a further RNA sample (extracted from a blood sample) obtained from the subject at a later time point;
(f) processing the RNA sample, optionally comprising synthesizing cDNA using the RNA as a template;
(g) sequencing the processed RNA or cDNA, wherein the sequencing output is used to identify the presence of an RNA (transcript) sequence associated with the disease, and
(h) using the identified RNA (transcript) sequence to produce a further RNA vaccine for the subject.

The RNA transcript may encode a protein isoform present in the subject with the disease. Using the identified RNA transcript sequence to produce the RNA vaccine may comprise producing an RNA molecule comprising at least a portion of the sequence, wherein the RNA molecule comprises an open reading frame (ORF), optionally encoding at least one antigenic peptide. The RNA molecule may further comprise a 5' UTR, 3' UTR, a polyA tail and/or a 5' cap. The 5' cap may have the Cap O structure or the Cap 1 structure. The Cap 0 structure may include a methyl-7 guanine nucleotide linked to the 5' position through a 5' triphosphate. The Cap 1 structure may be achieved by the methylation of the mRNA first nucleotide at the ribose 2'-O position. The RNA molecule may comprise one or two (optionally uridine-based) RNA strands, optionally with non-coding sequences optimised for translational performance. Thus, the RNA molecule in the RNA vaccine may comprise at least a portion of the wild-type sequence of the RNA transcript or may comprise a modified sequence. For example, the sequence may be adapted with respect to its codon usage. Adaption of codon usage can increase translation efficacy and half-life of the RNA. At least 25%, preferably at least 30%, 40%, 50%, 60%, 70%, 80%, 90%, 95% or even 100% of uridine present in the RNA sequence of each RNA molecule in the RNA vaccine may be replaced by pseudouridine or N1-methylpseudouridine or 15-methyluridine or 2-thiouridine. The RNA vaccine may comprise conventional (non-replicating) mRNA, self-amplifying mRNA and/or trans-amplifying mRNA (taRNA). Self-amplifying mRNA (saRNA) may be based on the addition of a viral replicase gene to enable the mRNA to self-replicate.

As used herein, the term "RNA vaccine" refers to a vaccine comprising an RNA molecule as defined herein. The vaccine may comprise, however, other substances and molecules which are required or which are advantageous when the vaccine is administered to an individual (e.g. pharmaceutical excipients). The RNA vaccine may comprise the RNA molecule in a buffer solution. The RNA molecule may be formulated in a lipid-based carrier, polymer or peptide, optionally a lipid nanoparticle. The RNA molecule may be formulated in a lipoplex nanoparticle comprising the synthetic cationic lipid (*R*)*-N,N,N-*trimethyl-2,3-dioleyloxy-1-propanaminium chloride (DOTMA) and the phospholipid 1,2-dioleoyl-sn-glycero-3-phosphatidylethanolamine (DOPE). The RNA molecule may be formulated to enable intravenous delivery. The RNA vaccine may be based on uridine mRNA-lipoplex nanoparticles (as described in Luis Rojas et al., Nature 2023; Jun 618(7963): 144-150 doi: 10.1038/s41586-023-06063-y, which is hereby incorporated by reference). The RNA vaccine may be delivered via transfection of dendritic cells. The RNA vaccine may encode more than 1, 2, 3, 4, 5, 6, 7, 8, 9, 10, 15 or 20 antigenic peptides.

The RNA vaccine may be manufactured as described in Sara Sousa Rosa et al. Vaccine. 2021 Apr 15;39(16):2190-2200 doi: 10.1016/j.vaccine.2021.03.038, which is hereby incorporated by reference.

The disease may be cancer or an infectious disease (e.g. COVID-19). The RNA vaccine may be an RNA cancer vaccine. The RNA transcript sequence may have a coding region that is cancer specific. The RNA transcript sequence may encode a protein isoform that is (uniquely) expressed in subjects with cancer. The set of RNA sequences in the transcript may encode neo-antigens (cancer specific peptide sub-sequences). The neo-antigens may be expressed on the cell surface via MHC complexes. Neoantigens may be produced by alternative splicing and/or RNA editing and may be predicted from RNA sequencing output (Jiyeon Park and Yeun-Jun Chung, Genomics and Informatics 2019;17(3):e23 DOI: https://doi.org/10.5808/G1.2019.17.3.e23 which is hereby incorporated by reference). The neoantigens may be targets for therapy (for example immunotherapy), optionally a cancer vaccine, adoptive cell therapy and/or antibody-based therapy (Na Xie et al., Signal Transduction and Targeted Therapy (2023)8:9 https://doi.org/10.1038/s41392-022-01270-x, which is hereby incorporated by reference). 2 or more, 5 or more, 10 or more or 15 or more neoantigens may be targeted by an RNA vaccine.

The invention provides an RNA vaccine for use in therapy, wherein the RNA vaccine is produced using the methods defined herein. A subject may receive an immune checkpoint inhibitor, optionally atezolizumab, prior to treatment with the RNA vaccine. The RNA vaccine may be administered to the subject 1, 2, 3, 4, 5, 6, 7, 8, or 9 or more times.

The invention provides a method for discovering a biomarker for a disease comprising:
(i) providing a (test) RNA sample obtained from a subject with the disease;
(ii) processing the RNA sample, optionally comprising synthesizing cDNA using the RNA as a template;
(iii) sequencing the processed RNA or cDNA; and
(iv) using the sequencing output to discover a disease biomarker.

The RNA sample may be extracted from a blood sample.

The invention provides a method for discovering a disease biomarker, the method comprising:
(a) providing a (test) RNA sample extracted from a blood sample obtained from a subject with the disease;
(b) processing the RNA sample, optionally comprising synthesizing cDNA using the RNA as a template; and
(c) sequencing the processed RNA or cDNA, wherein the sequencing output is used to discover a disease biomarker;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array;
   and
(ii) extracting the unannealed RNA or cDNA molecules thereby generating processed RNA or cDNA.

The method may further comprise:
(a) providing a control RNA sample extracted from a blood sample obtained from a subject without the disease;
(b) processing the control RNA sample, optionally comprising synthesizing cDNA using the RNA as a template; and
(c) sequencing the processed RNA or cDNA derived from the control RNA sample and comparing the sequencing output for the test RNA sample and control RNA sample to discover the disease biomarker.

Discovering a disease biomarker means identifying a novel biomarker (an indicator of a biological state) for a particular disease, for example uncovering a previously unknown biomarker for developing into a test for the disease. The disease biomarker may be suitable for use in diagnosing the disease, characterising the disease, predicting response to therapy, detecting minimal residual disease and/or prognosing the disease. By characterisation is meant classification and evaluation of the disease. Prognosis refers to predicting the likely outcome of the disease for the subject. The characterisation of and/or prognosis for the disease may comprise determining the grade and/or stage of the disease. The characterisation of the disease may comprise determining the sub-type of the disease. The disease biomarker may be suitable for use in indicating the likelihood that a subject with a particular disease will benefit from a specific therapy.

The disease may be cancer. The characterisation of and/or prognosis for the cancer may comprise determining the presence or absence of metastases. Metastasis, or metastatic disease, is the spread of a cancer from one organ or part to another non-adjacent organ or part. The new occurrences of disease thus generated are referred to as metastases. Characterisation of and/or prognosis for the disease may also comprise predicting biochemical recurrence and/or determining whether the cancer is aggressive and/or determining whether the cancer has spread to the lymph nodes. Aggressive refers to a cancer that is fast growing, more likely to spread, more likely to recur and/or shows resistance to treatment.

The invention provides a method for monitoring a subject comprising:
(i) providing a (test) RNA sample extracted from a blood sample obtained from the subject at a first time point and a further (test) RNA sample extracted from a blood sample from the subject at a second time point;
(ii) processing the first and the second RNA samples, optionally comprising synthesizing cDNA using the RNA as a template;
(iii) sequencing the processed RNA or cDNA; and
(iv) comparing the sequencing output for the test and further test samples.

Monitoring a subject may comprise monitoring response to treatment for a disease, for example monitoring whether treatment is successful and/or monitoring for adverse reactions/complications. The first time point may be prior to starting treatment and the second time point may be during or after treatment. Comparing the sequencing output for the test and further test RNA/cDNA samples may provide an indication as to whether treatment has been successful. For example, the presence or absence of a disease biomarker may indicate whether treatment has been successful. Comparing the sequencing output for the test and further test RNA/cDNA samples may comprise comparing to each other and/or to the sequencing output from a reference sample.

An immune system related transcript may be detected in the sequencing output. The first time point may be prior to starting treatment with an immunotherapy and the second time point may be during or after treatment with the immunotherapy.

The disease biomarker may be a cDNA sequence or an RNA sequence. The cDNA sequence will correspond to an RNA sequence. The cDNA/RNA sequence may correspond to a protein or peptide. The method may further comprise identifying an RNA, transcript, transcript model, gene, protein and/or peptide corresponding to a cDNA sequence. The disease biomarker may, therefore, be a cDNA molecule (of a specific sequence), DNA molecule (of a specific sequence), RNA molecule (of a specific sequence), transcript, transcript model, protein or peptide.

The method may comprise discovering more than one disease biomarker, optionally more than 10, 100, 1000, 10000, 100000, 1 million or 10 million disease biomarkers. The method may comprise discovering between 1 and 10, 1 and 100, 1 and 1000, 1 and 10000, 1 and 100000, 1 and 1 million or 1 and 10 million disease biomarkers.

Two or more of the disease biomarkers may be compiled to form a database. The invention provides a method for producing a database of disease biomarkers, the method comprising:
(i) providing two or more (test) RNA samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database.

The RNA samples may each be extracted from a blood sample. The invention provides a method for producing a database of disease biomarkers, the method comprising:
(i) providing two or more (test) RNA samples extracted from blood samples obtained from one or more subjects with a disease;
(ii) processing the RNA samples, optionally comprising synthesizing cDNA using the RNA as a template;
(iii) sequencing the processed RNA or cDNA, wherein the sequencing output is used to identify two or more disease biomarkers; and
(iv) compiling the disease biomarkers to form a database.

Each disease biomarker may be an RNA transcript, optionally wherein the RNA transcript encodes a protein isoform. The protein isoform may be present or absent in subjects with the disease.

The invention provides a method for producing a database of RNA sequences associated with a disease or condition, the method comprising:
(a) providing two or more (test) RNA samples obtained from one or more subjects;
(b) processing the RNA samples, optionally comprising synthesizing cDNA using the RNA as a template;
(c) sequencing the processed RNA or cDNA, wherein the sequencing output is used to identify the presence of two or more RNA transcript sequences associated with a disease or condition;
(d) identifying a set of RNA sequences in each RNA transcript; and
(e) compiling the sets of RNA sequences to form a database.

The RNA samples may each be extracted from a blood sample. The invention provides a method for producing a database of RNA sequences associated with a disease or condition, the method comprising:
(a) providing two or more (test) RNA samples extracted from blood samples obtained from one or more subjects;
(b) processing the RNA samples, optionally comprising synthesizing cDNA using the RNA as a template;
(c) sequencing the processed RNA or cDNA, wherein the sequencing output is used to identify the presence of two or more RNA transcript sequences associated with a disease or condition;
(d) identifying a set of RNA sequences in each RNA transcript; and
(e) compiling the sets of RNA sequences to form a database.

Each RNA sequence in the set may encode an antigenic peptide.

The sequencing output from a sample analysed and/or processed according to the methods defined herein may be compared to a database produced using a method defined herein in order to identify the presence of a disease biomarker or a set of RNA sequences associated with a disease or condition.

The database of disease biomarkers and the database of RNA sequences associated with a disease or condition may be used to guide treatment decisions and to aid in the development of new treatments. The disease biomarker and/or set of RNA sequences may be a suitable target for a therapeutic agent, for example a vaccine, an RNA therapy and/or gene editing. The discovery of a disease biomarker specific to cancer cells can be an initial step in identifying a cancer specific antigen for a cancer vaccine to target. Thus, the method may further comprise identifying a transcript or protein corresponding to the disease biomarker as a target for therapy, optionally a cancer vaccine target. The method may further comprise developing a therapy, for example a vaccine, optionally an RNA vaccine, directed to the target.

The methods may comprise providing 2, 3, 4, 5, 6, 7, 8, 9, 10, 20, 30, 40 or more RNA/cDNA samples from different subjects with the disease and/or providing 2, 3, 4, 5, 6, 7, 8, 9, 10, 20, 30, 40 or more RNA/cDNA samples from different subjects without the disease. Preferably, the methods comprise providing 30 or more RNA/cDNA samples from different subjects with the disease (e.g. with a cancer) and providing 30 or more RNA/cDNA samples from different subjects without the disease (e.g. without the cancer).

The methods may further comprise:
(a) extracting RNA from biological fluid (e.g. blood) or a fluid or lysate generated from a biological material from a subject with a disease and from a subject without the disease; and
(b) synthesizing cDNA using the RNA as a template (i.e. converting the RNA into cDNA).

A subject with a disease means the subject has the disease at the time the (biological) sample (biological fluid or biological material) from which the RNA/cDNA sample is derived is taken from the subject. A subject without a disease means the subject does not have the disease at the time the (biological) sample (biological fluid or biological material) from which the RNA/cDNA sample is derived is taken from the subject.

The subject without the disease may be a healthy subject.

The disease biomarker may be a cDNA molecule (of a specific sequence), RNA molecule (of a specific sequence), protein or peptide that is detectable in a sample from a subject with a disease but not in a sample from a subject without the disease. Alternatively, the disease biomarker may be a cDNA molecule (of a specific sequence), RNA molecule (of a specific sequence), protein or peptide that is not detectable in a sample from a subject with a disease but is detectable in a sample from a subject without the disease.

The cancer vaccine target may be a cDNA molecule (of a specific sequence), RNA molecule (of a specific sequence), protein or peptide that is detectable in a sample from a subject with a cancer but not in a sample from a subject without the cancer.

The disease biomarker may be a transcript that is (uniquely) present in subjects with a particular disease. In further embodiments, the disease biomarker is a transcript that is (uniquely) absent in subjects with a particular disease. In specific embodiments, the cancer vaccine target is a transcript that is uniquely present in subjects with a particular cancer. The transcript/RNA/cDNA sequence may correspond to a particular protein or peptide. At least a portion of the protein or peptide may form an antigen comprised in a cancer vaccine.

The present invention enables the identification of transcripts found only in subjects with a disease, optionally cancer. Such transcript models can be identified through comparison with subjects without the disease (e.g. benign patients) and, optionally, public transcriptome annotation databases. Sequencing output and/or resulting transcriptomic profile(s) from a subject with a particular disease (e.g. breast cancer) can be compared with sequencing output and/or resulting transcriptomic profile(s) from a subject with a different disease (for example, ovarian and/or colorectal cancer) to determine if the biomarker is unique to the particular disease (e.g. breast cancer).

The invention provides a method for diagnosing a disease in a subject comprising:
(i) providing a (test) RNA sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

The RNA sample may be extracted from a blood sample obtained from the subject.

The invention provides a method for diagnosing a disease or condition, wherein the method comprises:
(a) providing a (test) RNA sample obtained from a subject;
(b) processing the RNA sample, optionally comprising synthesizing cDNA using the RNA as a template;
(c) sequencing the processed RNA or cDNA, wherein the sequencing output is used to determine the presence or absence of a set of RNA sequences that are unique to an RNA transcript sequence that is only expressed in subjects with the disease or condition.

The RNA sample may be extracted from a blood sample. The invention provides a method for diagnosing a disease or condition, wherein the method comprises:
(a) providing a (test) RNA sample extracted from a blood sample obtained from a subject;
(b) processing the RNA sample, optionally comprising synthesizing cDNA using the RNA as a template;
(c) sequencing the processed RNA or cDNA, wherein the sequencing output is used to determine the presence or absence of a set of RNA sequences that are unique to an RNA transcript sequence that is only expressed in subjects with the disease or condition.

The disease may be an autoimmune disease, a cancer, diabetes, coronary disease, a metabolic disease, Alzheimer's disease, dementia, and/or an infectious disease. The disease may be a viral infection, bacterial infection and/or fungal infection. The disease may be COVID-19. The disease may be identified at a (very) early stage, optionally before symptoms have developed. The disease may be cancer and the cancer may be identified at a (very) early stage, optionally before significant tumour growth has occurred. The disease may be cancer (e.g. a hematological cancer such as leukemia, lymphoma or multiple myeloma) and diagnosing the disease may comprise detecting minimal residual disease (MRD). MRD may be defined as cancer cells that remain in the subject during or after treatment.

The invention provides a method for diagnosing cancer in a subject, the method comprising:
(a) providing a (test) RNA sample extracted from a blood sample obtained from the subject;
(b) processing the RNA sample, optionally comprising synthesizing cDNA using the RNA as a template; and
(c) sequencing the processed RNA or cDNA, wherein the sequencing output is used to identify whether the subject has cancer;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array;
   and
(ii) extracting the unannealed RNA or cDNA molecules thereby generating processed RNA or cDNA.

The sequencing output may be used to determine the presence or absence of one or more RNA molecules/sequences/transcripts in the RNA sample in order to identify whether the subject has cancer.

The disease may be an autoimmune disease.

The invention provides a method for diagnosing an autoimmune disease in a subject comprising:
(i) providing a (test) RNA sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

The RNA sample may be extracted from a blood sample.

The invention provides a method for diagnosing an autoimmune disease in a subject comprising:
(i) providing a (test) RNA sample extracted from a blood sample obtained from the subject;
(ii) processing the RNA sample, optionally comprising synthesizing cDNA using the RNA as a template; and
(iii) sequencing the processed RNA or cDNA, wherein the sequencing output is used to identify whether the subject has the disease.

An "autoimmune disease" herein is a disease or disorder wherein the immune system of a subject mounts an immune response to the subject's own tissue. The autoimmune disease may be arthritis, celiac disease, diabetes mellitus type 1, graves' disease, inflammatory bowel disease, multiple sclerosis, alopecia areata, Addison's disease, pernicious anemia, psoriasis, systemic lupus erythematosus, myasthenia gravis, Hashimoto's thyroiditis, Vitiligo, Sjogren's syndrome, myositis, chronic inflammatory demyelinating polyneuropathy (CIDP), dermatomyositis, Guillain-Barre syndrome, ulcerative colitis, Crohn's disease and/or vasculitis.

The sequencing output may be used to identify whether the subject has the disease by comparing to a database of biomarkers, optionally wherein the database of biomarkers is produced using a method defined herein.

By diagnosing is meant determining that a subject has the disease at the time of testing.

The methods defined herein may further comprise selecting a treatment appropriate for the disease and, optionally, treating the disease with the selected treatment. Treating the subject may start at an early stage of disease progression, optionally before symptoms have appeared or significant tumour growth has occurred.

The invention provides a method for characterising and/or prognosing a disease in a subject comprising:
(i) providing a test RNA/cDNA sample from the subject;
(ii) processing (normalizing) the RNA/cDNA sample; and
(iii) sequencing the processed (normalized) RNA/cDNA sample, wherein the sequencing output is used to provide a characterisation of and/or a prognosis for the disease.

The invention provides a method for selecting a treatment for a disease in a subject comprising:
(i) providing a (test) RNA/cDNA sample from the subject;
(ii) processing (normalizing) the RNA/cDNA sample;
(iii) sequencing the processed (normalized) RNA/cDNA sample, wherein the sequencing output is used to provide a diagnosis, characterisation of and/or a prognosis for the disease; and
(iv) selecting a treatment appropriate to the diagnosis, characterisation of and/or prognosis for the disease.

The invention provides a method for predicting the responsiveness of a subject with a disease to a therapeutic agent comprising:
(i) providing a test RNA/cDNA sample from the subject;
(ii) processing (normalizing) the RNA/cDNA sample; and
(iii) sequencing the processed (normalized) RNA/cDNA sample, wherein the sequencing output is used to predict the responsiveness of the subject to the therapeutic agent.

The therapeutic agent may be an immune checkpoint inhibitor and/or immunotherapy, optionally CAR-T therapy. The RNA/cDNA sample may comprise full-length RNA/cDNA and/or the processed RNA/cDNA sample comprises full-length RNA/cDNA.

The methods as described herein may further comprise treating the subject.

The methods may comprise comparing the sequencing output for the processed (normalized) RNA/cDNA sample to one or more reference sequences or to the sequencing output of one or more control samples, optionally wherein the one or more control samples are from one or more subjects with and/or without the disease. Preferably, the methods comprise comparing the sequencing output for the processed (normalized) RNA/cDNA sample to the sequencing output of one or more control samples from one or more subjects with the disease.

By sequencing output is meant one or more sequences obtained from sequencing the processed (normalized) RNA/cDNA. The sequence(s) may be raw sequence(s) or may be further processed. For example, low quality reads may be filtered and/or adapter sequences may be filtered and removed. The (processed) sequence(s) may be mapped to the human reference genome (for example, using Minimap2) to prepare transcriptome profile(s). One or more transcript models may be identified in the transcriptome profile(s) (sequence(s) mapped to the genome). The transcript model represents a specific transcript i.e. a particular RNA isoform or splice variant produced from a gene. Thus, in specific embodiments, the sequencing output that is used in the methods defined herein (for example, that is compared to discover a disease biomarker or is used to identify whether the subject has a disease) may be transcript(s), transcriptome profile(s) and/or transcript model(s).

Using the sequencing output to identify whether the subject has the disease may comprise detecting a disease biomarker. Using the sequencing output to identify whether the subject has the disease may comprise detecting more than one disease biomarker, optionally more than 10, 100, 1000, 10000, 100000, 1 million or 10 million disease biomarkers. Using the sequencing output to identify whether the subject has the disease may comprise detecting between 1 and 10, 1 and 100, 1 and 1000, 1 and 10000, 1 and 100000, 1 and 1 million or 1 and 10 million disease biomarkers. Detecting the disease biomarker may comprise determining the presence or absence of the disease biomarker. Using the sequencing output to identify whether the subject has the disease may comprise determining the presence or absence of more than one disease biomarker, optionally more than 10, 100, 1000, 10000, 100000, 1 million or 10 million disease biomarkers. Using the sequencing output to identify whether the subject has the disease may comprise determining the presence or absence of between 1 and 10, 1 and 100, 1 and 1000, 1 and 10000, 1 and 100000, 1 and 1 million or 1 and 10 million disease biomarkers.

The presence of a particular RNA/cDNA sequence in the sequencing output may indicate that the subject has the disease, for example where a particular transcript (corresponding to the cDNA molecule) is uniquely present in subjects with a particular disease. Likewise, the presence of a particular RNA/cDNA sequence in the sequencing output may indicate a characterisation of and/or a prognosis for the disease. The presence of a particular RNA/cDNA sequence in the sequencing output may allow prediction of the responsiveness of a subject with a disease to a therapeutic agent, for example where a particular transcript has been found to correlate with responsiveness of a subject with a disease to a particular therapeutic agent.

The absence of a particular RNA/cDNA sequence in the sequencing output may indicate that the subject has the disease, for example where a particular transcript (corresponding to the cDNA molecule) is absent in subjects with a particular disease. Likewise, the absence of a particular RNA/cDNA sequence in the sequencing output may indicate a characterisation of and/or a prognosis for the disease. The absence of a particular RNA/cDNA sequence in the sequencing output may allow prediction of the responsiveness of a subject with a disease to a therapeutic agent, for example where a particular transcript has been found to correlate with responsiveness of a subject with a disease to a particular therapeutic agent.

RNA samples may be obtained from biological samples of any suitable form including any material, biological fluid, tissue, or cell obtained or otherwise derived from a subject. The sample may include cancer cells or genetic material (DNA or RNA) derived from the cancer cells, to include cell-free genetic material (e.g. found in the peripheral blood). The sample may comprise a biopsy sample (e.g. a formalin-fixed paraffin-embedded biopsy sample). The sample may comprise a fresh/frozen (FF) sample. The sample may comprise tumour (cancer) tissue, optionally breast tumour (cancer) tissue. The sample may comprise tumour (cancer) cells, optionally breast tumour (cancer) cells. The tissue sample may be obtained by any suitable technique. Examples include a biopsy procedure, optionally a fine needle aspirate biopsy procedure. Body fluid samples may also be utilised. Suitable sample types include blood (including whole blood, leukocytes, peripheral blood mononuclear cells, buffy coat, plasma, and serum), sputum, tears, mucus, nasal washes, nasal aspirate, breath, urine, semen, saliva, meningeal fluid, amniotic fluid, glandular fluid, lymph fluid, nipple aspirate, bronchial aspirate, synovial fluid, joint aspirate, ascites, cells, a cellular extract, and cerebrospinal fluid. This also includes experimentally separated fractions of all of the preceding. For example, a blood sample can be fractionated into serum or into fractions containing particular types of blood cells, such as red blood cells or white blood cells (leukocytes). If desired, a sample can be a combination of samples from a subject, such as a combination of a tissue and fluid sample. The term "sample" also includes materials containing homogenized solid material, such as from a stool sample, a tissue sample, or a tissue biopsy, for example. The term "sample" also includes materials derived from a tissue culture or a cell culture, including tissue resection and biopsy samples. Example methods for obtaining a sample include, e.g., phlebotomy, swab (e.g., buccal swab). Samples can also be collected, e.g., by micro dissection (e.g., laser capture micro dissection (LCM) or laser micro dissection (LMD)), bladder wash, smear (e.g., a PAP smear), or ductal lavage. A "sample" obtained or derived from a subject includes any such sample that has been processed in any suitable manner after being obtained from the subject. The methods of the invention as defined herein may begin with an obtained sample and thus do not necessarily incorporate the step of obtaining the sample from the patient.

The RNA/cDNA sample may be obtained from a tumour (e.g. a solid biopsy) or from biological fluid or a fluid or lysate generated from a biological material. The RNA/cDNA sample may be obtained from blood. The RNA/cDNA sample may be obtained by extracting RNA from a biological sample (e.g. blood) obtained from the subject. cDNA is then synthesized using the RNA as a template (i.e. by reverse transcription). Thus, the methods may further comprise:
(a) extracting RNA from biological fluid (e.g. blood) or a fluid or lysate generated from a biological material from the subject; and/or
(b) synthesizing cDNA using the RNA as a template (i.e. converting the RNA into cDNA).

In this way the cDNA sample from the subject may be produced.

The methods may further comprise reporting to the subject the outcome of the method. The result may be a diagnosis or prognosis for the disease. The result may be a specific grade or stage of a disease, such as a cancer.

The term "sequence" may refer to all of the individual nucleic acid (e.g. cDNA or RNA) molecules having a 100% identical nucleotide sequence. Alternatively, the term "sequence" may refer to all of the individual nucleic acid (e.g. cDNA or RNA) molecules having more than 99%, 98%, 97%, 96%, 95%, 90%, 85%, 80%, 75%, 70%, 65%, 60%, 55% or 50% identity to one another. "% identity" between a query nucleic acid sequence and a subject nucleic acid sequence may be calculated using a suitable algorithm (e.g. BLASTN, FASTA, Needleman-Wunsch, Smith-Waterman, LALIGN, or GenePAST/KERR) or software (e.g. DNASTAR Lasergene, GenomeQuest, EMBOSS needle or EMBOSS infoalign), over the entire length of the query sequence after a pair-wise global sequence alignment has been performed using a suitable algorithm (e.g. Needleman-Wunsch or GenePAST/KERR) or software (e.g. DNASTAR Lasergene or GenePAST/KERR). The term "unique sequence" or "unique cDNA sequence" or "unique RNA sequence" may refer to all of the individual nucleic acid (e.g. cDNA or RNA as appropriate) molecules which meet or exceed a threshold % identity (e.g. 100%, 99%, 98%, 97%, 96%, 95%, 90%, 85%, 80%, 75%, 70%, 65%, 60%, 55% or 50% identity to one another). The "unique sequence" or "unique cDNA sequence" or "unique RNA sequence" may differ from the other sequences present in the sample (for example, by at least 1, 2, 3, 4, 5, 6, 7, 8, 9, 10, 20, 50 or 100 nucleotides or by at least 1%, 2%, 3%, 4%, 5%, 10%, 15%, 20%, 25%, 30%, 35%, 40%, 45% or 50% of their sequence).

The disease may not be an infectious disease. The disease may be cancer. The cancer may be an epithelial cancer. The cancer may be breast, ovarian and/or colorectal cancer. Preferably, the cancer is breast cancer.

The methods may be used to diagnose more than one disease in a single process, for example through detection of multiple RNA or cDNA molecules (derived from transcripts) that are each uniquely present in subjects with a particular disease. The methods may be used to diagnose more than one autoimmune disease.

The methods may be used to diagnose more than one cancer type. The methods may be used to distinguish between breast cancer and a benign breast condition.

Sequencing may comprise the use of long read sequencing. By using long read sequencing, it is possible to detect full length RNA/cDNA which will provide better information for identifying the tissue source of each RNA and the specific function.

Sequencing may comprise long read sequencing. Long read sequencing may be single-molecule long read sequencing (e.g. PacBio^{®} HiFi or Oxford Nanopore Technologies nanopore sequencing). Long read sequencing may be single-molecule nanopore sequencing. Long read sequencing may comprise tagmentation (e.g. Ilumina Complete Long Read sequencing technology). Long read sequencing may produce reads of more than 1 kb, more than 5kb, more than 10kp or more than 20kb.

The RNA may be full-length RNA. Thus, the processed RNA or cDNA that is sequenced may be full length. By "full length" is meant that the sequence of the whole length (or at least 99%, 98%, 95%, 90% or 80% of the length) of the RNA/cDNA molecule may be obtained i.e. RNA or cDNA molecules are not fragmented before sequencing. Entire spliced isoforms may be directly observed. Thus, the methods defined herein may not comprise a step of (actively) fragmenting RNA and/or cDNA prior to sequencing.

Sequencing may comprise the use of long-read, full-length RNA sequencing. This allows for direct observation of entire spliced isoforms.

The cDNA sample may comprise no more than 800ng, 700ng, 500ng, 100ng, 20ng, 10ng, 5ng or 1ng of starting cDNA. The cDNA sample may comprise 1-800 ng, 1-500ng, 5-100ng, or 10-50ng of starting cDNA.

RNA from a sample may be firstly reverse transcribed to cDNA. Sample types include blood samples (in particular from plasma, and also serum), other bodily fluids such as saliva, urine or lymph fluid. Other sample types include solid tissues, including frozen tissue or formalin fixed, paraffin embedded (FFPE) material. The RNA may be messenger RNA (mRNA), microRNA (miRNA) etc. The RNA may be reverse transcribed using a reverse transcriptase enzyme to form a complementary DNA (cDNA) molecule. Methods for reverse transcribing RNA to cDNA using a reverse transcriptase are well-known in the art. Any suitable reverse transcriptase can be used, examples of suitable reverse transcriptases being widely available in the art. The initial cDNA molecule may be single stranded until DNA polymerase has been used to generate the complementary strand. Commercially available kits (such as NEBNext ^{®} Single Cell/Low Input cDNA Synthesis & Amplification Module) can be used to convert RNA into double stranded cDNA with 5' and 3' adapters. Primers based on the 5' and 3' adapters can be used to add phosphate groups to the cDNA. A cDNA purification step (for example with ProNex or Ampure beads) may be carried out prior to use of the cDNA as a cDNA sample or prior to sequencing.

As the present invention only requires a low amount of starting cDNA, this can be produced from a small quantity of RNA and/or without the requirement for additional PCR cycles during the generation of the cDNA. The RNA sample may comprise no more than 3.5µg, 3µg, 2µg, 1µg, 500ng, 100 ng, 10ng or 1ng of starting RNA. The RNA sample may comprise 1ng-3µg, 10ng-2µg, or 100ng-1µg of starting RNA.

Processing the RNA sample may comprise normalization (reducing the variability in the levels of different RNA or cDNA sequences in the sample). Thus, the processed RNA or cDNA sample may be a normalized RNA or cDNA sample.

Processing the RNA sample may comprise equalizing the sample. Thus, in the processed RNA or cDNA the relative abundance of all the unique RNA or cDNA sequences may be more equal. For example, the levels of the unique sequences in the processed RNA or cDNA sample may vary by less than 50%, less than 40%, less than 30%, less than 20%, or less than 10%.

Processing an RNA or cDNA sample may reduce the variability in the levels of the RNA or cDNA (e.g. by at least 10%, at least 20%, at least 30%, at least 40%, at least 50%, at least 60%, at least 70%, at least 80%, or at least 90%). Processing RNA or cDNA may achieve a more uniform distribution of cDNA sequences. The difference in abundance between the most abundant RNA/cDNA and the least abundant RNA/cDNA in the sample may be reduced (e.g. by at least 10%, at least 20%, at least 30%, at least 40%, at least 50%, at least 60%, at least 70%, at least 80%, or at least 90%). Processing the RNA or cDNA sample may reduce the number of molecules (copy number) of the (1, 10, 100, 1000, or 10000) most abundant RNA or cDNA molecule(s) by at least 10%, at least 20%, at least 30%, at least 40%, at least 50%, at least 60%, at least 70%, at least 80%, or at least 90%. The number of molecules (copy number) of the most abundant RNA or cDNA molecule in the (first and/or second) RNA or cDNA sample may be reduced by at least 50% in the processed RNA or cDNA. The relative abundance of the (1, 10, 100, 1000, or 10000) least abundant RNA or cDNA molecule(s) may be increased by at least 10%, at least 20%, at least 30%, at least 40%, at least 50%, at least 60%, at least 70%, at least 80%, or at least 90%. The number of molecules (copy number) of the least abundant RNA or cDNA molecule in the (first and/or second) cDNA sample may be increased by at least 50% in the processed RNA or cDNA.

The processed RNA or cDNA sample may be more readily analysable. It may be more efficiently sequenced because the relative representation or levels of less abundant sequences is increased.

Normalizing an RNA or cDNA sample results in production of a normalized RNA or cDNA sample. Normalizing may comprise (selectively) increasing the relative abundance of less abundant sequences without targeting specific sequences based on their nucleotide sequence (i.e. identity or homology to a known sequence).

By "normalized" is meant that the levels of RNA or cDNA sequences in the sample are more equal. Thus, a normalized RNA or cDNA sample may be one in which the amount of each unique RNA or cDNA sequence is more uniform than in the same sample prior to normalization i.e. a normalized RNA or cDNA sample is closer to achieving each unique RNA or cDNA sequence having the same abundance (relative to other unique RNA or cDNA sequences within the normalized RNA or cDNA sample) than the same sample prior to normalization. To achieve this the relative representation or levels of less abundant sequences may be increased and/or the relative representation or levels of more abundant sequences may be decreased. The increase in less abundant sequences/decrease in more abundant sequences is selective in the sense that if all sequences were increased/decreased to the same degree the relative abundance would stay the same. However, the relative representation or levels of less abundant sequences may be increased and/or the relative representation or levels of more abundant sequences may be decreased without targeting (for example, using pre-defined probes) specific sequences based on their nucleotide composition (i.e. based on their identity or homology to a known sequence). The less abundant sequences may be the unique sequences with an amount that is below a threshold, for example they are present in the RNA or cDNA sample prior to normalization in an amount that is below the mean amount for a unique sequence in the sample. The less abundant sequences may be present in the RNA or cDNA sample prior to normalization at an amount that is 0.1 %, 1%, 10%, 20%, 30%, 40%, 50%, 60%, 70, 80% or 90% below the mean amount for a unique sequence in the sample. The more abundant sequences may be the unique sequences with an amount that is above a threshold, for example they are present in the RNA or cDNA sample prior to normalization in an amount that is above the mean amount for a unique sequence in the sample. The more abundant sequences may be present in the RNA or cDNA sample prior to normalization at an amount that is 0.1 %, 1%, 10%, 20%, 30%, 40%, 50%, 60%, 70, 80% or 90% above the mean amount for a unique sequence in the sample. By relative abundance is meant abundance relative to other unique sequences in the sample.

A normalized RNA or cDNA sample may comprise RNA or cDNA sequences having substantially the same levels. For example, wherein the levels of the sequences of the normalized RNA or cDNA vary by less than 50%, less than 40%, less than 30%, less than 20%, or less than 10%. The normalized RNA or cDNA may be a normalized RNA or cDNA sample in which at least a portion of the 10, 100, 1000, or 10000 most abundant (unique) sequences in the RNA or cDNA sample have been removed or reduced (by at least 1%, 10%, 20%, 30%, 40%, 50%, 60%, 70%, 80% or 90%) in copy number. The normalized RNA or cDNA may be a normalized RNA or cDNA sample in which levels of at least a portion of the 10, 100, 1000, or 10000 least abundant (unique) sequences in the RNA or cDNA sample have been increased e.g. by at least 1%, 10%, 20%, 30%, 40%, 50%, 60%, 70%, 80% or 90% in copy number. The methods for normalizing RNA or cDNA sample(s) described herein may be methods for equalizing cDNA sample(s) i.e. equalizing the relative abundances of each unique sequence.

Normalizing the RNA or cDNA sample(s) may increase the amount (copy number) of at least a portion of the low abundance RNA or cDNA sequences within the (first and/or the second) cDNA sample(s). The low abundance RNA or cDNA sequences may be the 50%, 40%, 30%, 20%, 10% or 1% of (unique) sequences with the lowest copy number. Thus, normalizing may comprise selectively increasing the amount of low abundance RNA or cDNA within each RNA or cDNA sample.

Normalized RNA or cDNA may be RNA or cDNA that is more readily analysable. It may be more efficiently sequenced because the relative representation of less abundant sequences is increased. Normalizing the RNA or cDNA sample(s) may not comprise removing abundant (more abundant) RNA or cDNA molecules/sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin) from the sample(s) (for example, using duplex-specific nuclease or sequence targeted methods). Normalizing may not comprise targeting specific (unique) sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin). Normalizing a RNA or cDNA sample may be non-targeted i.e. it may not involve targeting specific sequences based on their nucleotide sequence (for example, it may not involve targeting a particular sequence based on its identity or homology to a known sequence).

Processing the RNA sample(s) may improve detection of low-abundance RNA or cDNA, optionally wherein processing the RNA sample(s) comprises increasing the amount of low abundance RNA or cDNA within each sample.

Methods that may be used for processing an RNA or cDNA sample include depletion methods (such as CRISPR-based depletion methods), methods that comprise inhibiting reverse transcription of abundant RNA sequences (e.g. inhibition of cDNA synthesis using oligo blockers) and normalization (e.g. cDNA or RNA normalization) methods.

Depletion methods comprise removing unwanted RNA or cDNA molecules/sequences. These may be contaminating RNA or cDNA molecules/sequences (for example bacterial transcripts, optionally bacterial ribosomal RNA) or abundant (more abundant) RNA or cDNA molecules/sequences (such as those corresponding to ribosomal, mitochondrial, globin and housekeeping genes, optionally those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin) from the sample(s). CRISPR-Cas9 may be used to degrade abundant sequences. Optionally, CRISPR-Cas9 complexes are formed with a pool of designed guide RNAs, and the complexes are mixed with a cDNA sample. After the unwanted abundant sequences are cut, they cannot be substrates for PCR amplification and subsequent sequencing. Example products include CRISPclean^{™} Stranded Total RNA Prep with rRNA Depletion from Jumpcode Genomics.

Methods that comprise inhibition of reverse transcription may use high-affinity RNA-binding oligonucleotides to block reverse transcription and/or PCR amplification of specific RNA transcripts (see, for example, Everaert C et al., Biological Procedures Online 25, Article number: 7 (2023) https://doi.org/10.1186/s12575-023-00193-3, which is hereby incorporated by reference). An LNA-modified oligonucleotide complementary to an unwanted RNA can be designed, which can block reverse transcription and/or PCR amplification when bound downstream of the priming site.

Complementary DNA (cDNA) normalization (Alex S. Shcheglov, Pavel A. Zhulidov, Ekaterina A. Bogdanova, D. A. S. Normalization of cDNA Libraries, Nucleic Acids Hybrid. CHAPTER 5, (2014)) addresses issues with high abundance house-keeping genes reducing sampling efficiency for genes of interest. Since RNA sequencing typically relies on the conversion of RNA to double stranded cDNA, cDNA normalization takes advantage of the biochemical properties of cDNA to generate a uniform distribution of unique genes and isoforms within a cDNA library. In theory, the maximum non-targeted sampling efficiency is produced if all unique RNA sequences are represented at the same relative abundance. Thus, the objective of normalization is to re-distribute a cDNA library (sample) to meet this criterion as closely as possible.

Complementary DNA (cDNA) normalization may be full length cDNA normalization. Complementary DNA (cDNA) normalization may be performed by the Duplex Specific Nuclease (DSN) method (see e.g. Zhulidov, P. A. et al. Simple cDNA normalization using kamchatka crab duplex-specific nuclease. Nucleic Acids Res. 32, e37 (2004)) or the hydroxyapatite column method (see e.g. Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010) which is hereby incorporated by reference). Both methods rely on the denaturation and re-hybridization of cDNA strands. As the single stranded cDNA move about in solution, the sequences that are more highly abundant have a greater probability of finding a matching complementary sequence with which to re-hybridize. Thus, as re-hybridization reaches its limit, the remaining single stranded cDNA represents a normalized sequence library.

Thus, processing the RNA sample may comprise synthesizing double stranded cDNA using the RNA as a template and then denaturing and re-hybridizing the cDNA strands.

The difference between the DSN method and the hydroxyapatite column method lies in their approach for isolating the single stranded cDNA library from the re-hybridized double stranded cDNA molecules.

In the DSN method, an enzyme which specifically cleaves double stranded DNA is used to decompose all double stranded cDNA within the solution. The solution is then purified and size-selected for cDNA sequences above a certain length. These sequences are then amplified using the Polymerase Chain Reaction (PCR).

In the column method, the denatured and re-hybridized cDNA library is passed through a heated column filled with hydroxyapatite granules. The hydroxyapatite preferentially binds to larger DNA molecules. The size of DNA that is bound is controlled by the concentration of phosphate buffer in which the cDNA library is dissolved. Thus the concentration of phosphate buffer must be tuned specifically for cDNA molecules within a certain range of sequence length. The cDNA is eluted through the column using increasing concentrations of phosphate buffer to extract increasing sizes of DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

The invention provides methods and devices for preparing processed nucleic acid samples with a more uniform distribution of sequences, including methods for RNA and cDNA normalization. A first nucleic acid sample is used to produce a probe set based on the intrinsic sequence abundances in the sample. Abundant sequences will produce more probes. When a second nucleic acid sample is applied to the probes more of the abundant sequences will bind to the probes enabling these sequences to be separated from the sample. In this manner the present invention enables normalization of full-length RNA, as well as cDNA.

The method for processing nucleic acid may comprise:
(i) contacting a nucleic acid sample (e.g. an RNA sample) with an oligonucleotide array (e.g. a DNA, optionally a cDNA array), wherein one or more nucleic acid molecules from the nucleic acid sample anneal to one or more oligonucleotides of the oligonucleotide array; and
(ii) extracting the unannealed nucleic acid molecules thereby generating processed nucleic acid.

Processing the RNA or cDNA sample may comprise:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

The array may be produced by a method comprising:
(i) bringing an RNA sample into contact with a surface, wherein the surface comprises two or more oligo-dT molecules, and wherein two or more RNA molecules from the RNA sample anneal to the oligo-dT molecules;
(ii) extending two or more of the oligo-dT molecules by reverse transcription using the annealed RNA molecules as templates to generate a DNA array comprising two or more cDNA molecules;
(iii) disassociating the annealed RNA molecules from the cDNA molecules;
(iv) removing the RNA sample from the surface.

The array may comprise two or more oligonucleotides with sequences comprising oligo-dT followed by a cDNA sequence.

The DNA array may be produced by a method comprising:
(i) contacting a preparatory RNA sample with an oligonucleotide array, wherein the oligonucleotide array comprises two or more oligonucleotides linked to a surface, and wherein two or more RNA molecules from the RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the RNA sample from the surface.

The preparatory RNA sample and the test RNA sample may be derived from the same subject, optionally wherein the preparatory RNA sample and the test RNA sample are derived from the same blood sample.

The array may be produced by a method comprising:
(i) denaturing a cDNA sample comprising double stranded cDNA molecules to produce single stranded cDNA molecules;
(ii) bringing the cDNA sample into contact with a surface, wherein the surface comprises two or more oligo-dT molecules, and wherein two or more cDNA molecules from the cDNA sample anneal to the oligo-dT molecules;
(iii) extending two or more of the oligo-dT molecules using the annealed cDNA molecules as templates to generate a DNA array comprising two or more DNA molecules;
(iv) disassociating the annealed cDNA molecules from the DNA molecules;
(v) removing the cDNA sample from the surface.

The array may comprise two or more oligonucleotides with sequences comprising oligo-dT followed by a DNA sequence.

The method for processing nucleic acid may comprise:
(i) contacting a first nucleic acid sample with an oligonucleotide array, wherein the oligonucleotide array comprises two or more oligonucleotides linked to a surface, and wherein two or more nucleic acid molecules from the first nucleic acid sample anneal to the oligonucleotides of the oligonucleotide array;
(ii) extending two or more of the oligonucleotides using the annealed nucleic acid molecules as templates to generate a DNA array comprising two or more DNA molecules;
(iii) disassociating the annealed nucleic acid molecules from the DNA molecules;
(iv) removing the first nucleic acid sample from the surface;
(v) contacting a second nucleic acid sample with the DNA array, wherein one or more nucleic acid molecules from the second nucleic acid sample anneal to the DNA molecules; and
extracting the unannealed nucleic acid molecules thereby generating processed nucleic acid.

The nucleic acid is not limiting according to the invention. Any suitable nucleic acid molecule may processed using the devices, kits and methods of the invention. The nucleic acid may be double stranded or single stranded, optionally when the nucleic acid molecules are double-stranded, the double stranded nucleic acid molecules are first denatured to produce single stranded nucleic acid molecules.

The nucleic acid may be DNA. The DNA may be genomic DNA, mitochondrial DNA, cDNA etc. cDNA is preferred. The DNA may be purified from any suitable sample. Sample types include blood samples (in particular from plasma, and also serum), other bodily fluids such as saliva, urine or lymph fluid. Other sample types include solid tissues, including frozen tissue or formalin fixed, paraffin embedded (FFPE) material. The DNA molecule may be a double-stranded DNA (dsDNA) molecule. The DNA molecule may be a single-stranded DNA (ssDNA) molecule. ssDNA may already have been denatured *in situ* in the original sample. For example, the ssDNA may be purified from FFPE material. The nucleic acid sample may comprise both ssDNA and dsDNA molecules. For instance, in the case of DNA purified from FFPE material, the DNA may include both ssDNA and dsDNA. The DNA may be found in, or derived from cells in a sample. Alternatively the DNA may be circulating, or "cell-free", DNA (cfDNA). Such DNA can be obtained from a range of bodily fluids including blood samples (in particular from plasma, and also serum), other bodily fluids such as saliva, urine or lymph fluid.

The nucleic acid may also be RNA. RNA may be obtained from the same sample types as DNA, as discussed above. The RNA may be messenger RNA (mRNA), microRNA (miRNA), transfer RNA (tRNA), ribosomal RNA (rRNA), long non-coding RNA (IncRNA), small interfering RNA (siRNA), small nucleolar RNA (snoRNA), piwi-interacting RNA (piRNA), tRNA-derived small RNA (tsRNA), small rDNA-derived RNA (srRNA), viral RNA etc. mRNA is preferred.

Processing the RNA sample may comprise:
(i) contacting a first RNA sample with an oligonucleotide array, wherein the oligonucleotide array comprises two or more oligonucleotides linked to a surface, and wherein two or more RNA molecules from the first RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the first RNA sample from the surface;
(v) contacting a second RNA sample with the DNA array, wherein one or more RNA molecules from the second RNA sample anneal to the cDNA molecules; and
(vi) extracting the unannealed RNA molecules thereby generating processed RNA.

The first RNA sample may also be referred to as a preparatory RNA sample. The second RNA sample may also be referred to as a test RNA sample. The RNA sample to be processed may be split to form the first/preparatory RNA sample and second/test RNA sample.

The invention provides a method for determining a set of RNA sequences associated with a disease or condition, the method comprising:
(a) providing a test RNA sample extracted from a blood sample obtained from a subject;
(b) processing the test RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript;

wherein processing the test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The invention provides a method for producing a database of RNA sequences associated with a disease or condition, the method comprising:
(a) providing two or more test RNA samples extracted from blood samples obtained from one or more subjects;
(b) processing the test RNA samples, optionally comprising synthesizing cDNA using the RNA as a template;
(c) sequencing the processed RNA or cDNA, wherein the sequencing output is used to identify the presence of two or more RNA transcript sequences associated with a disease or condition;
(d) identifying a set of RNA sequences in each RNA transcript; and
(e) compiling the sets of RNA sequences to form a database;

wherein processing each test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The invention provides a method for diagnosing a disease or condition, wherein the method comprises:
(a) providing a test RNA sample extracted from a blood sample obtained from a subject;
(b) processing the test RNA sample, optionally comprising synthesizing cDNA using the RNA as a template;
(c) sequencing the processed RNA or cDNA, wherein the sequencing output is used to determine the presence or absence of a set of RNA sequences that are unique to an RNA transcript sequence that is only expressed in subjects with the disease or condition;

wherein processing the test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The invention provides a method for producing an RNA vaccine for a subject with a disease, the method comprising:
(a) providing a test RNA sample extracted from a blood sample obtained from the subject;
(b) processing the test RNA sample, optionally comprising synthesizing cDNA using the RNA as a template;
(c) sequencing the processed RNA or cDNA, wherein the sequencing output is used to identify the presence of an RNA transcript sequence associated with the disease; and
(d) using the identified RNA transcript sequence to produce a first RNA vaccine for the subject;

wherein processing the test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The invention provides a method for discovering a disease biomarker, the method comprising:
(a) providing a test RNA sample extracted from a blood sample obtained from a subject with the disease;
(b) processing the test RNA sample, optionally comprising synthesizing cDNA using the RNA as a template; and
(c) sequencing the processed RNA or cDNA, wherein the sequencing output is used to discover a disease biomarker;

wherein processing the test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The invention provides a method for diagnosing cancer in a subject, the method comprising:
(a) providing a test RNA sample extracted from a blood sample obtained from the subject;
(b) processing the test RNA sample, optionally comprising synthesizing cDNA using the RNA as a template; and
(c) sequencing the processed RNA or cDNA, wherein the sequencing output is used to identify whether the subject has cancer;

wherein processing the test RNA or cDNA sample comprises:
   (i) contacting the test RNA or cDNA sample with a DNA array, comprising two or more DNA molecules, wherein one or more RNA or cDNA molecules from the test RNA or cDNA sample anneal to the DNA molecules of the DNA array; and
   (ii) extracting the unannealed RNA or cDNA molecules thereby generating processed RNA or cDNA;
and wherein the DNA array was produced by a method comprising:
   (i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
   (ii) extending two or more of the oligonucleotides by reverse transcription using the annealed RNA molecules as templates to generate a DNA array comprising two or more DNA (cDNA) molecules;
   (iii) disassociating the annealed RNA molecules from the DNA (cDNA) molecules; and
   (iv) removing the preparatory RNA sample from the surface.

The preparatory RNA sample may be extracted from a blood sample obtained from a subject, optionally the same subject as the test RNA sample. The preparatory RNA sample and test RNA sample may be derived from the same blood sample. Where there is more than one test RNA sample, each test RNA sample may have a corresponding preparatory RNA sample derived from the same blood sample. The methods defined herein may further comprise steps of providing a blood sample obtained from a subject and extracting a preparatory RNA sample and a test RNA sample from the blood sample.

The invention provides use of a method for processing a test RNA or cDNA sample in a method for determining soil microfauna composition, wherein the method for processing an RNA or cDNA sample comprises:
(i) contacting the test RNA or cDNA sample with a DNA array, comprising two or more DNA molecules, wherein one or more RNA or cDNA molecules from the test RNA or cDNA sample anneal to the DNA molecules of the DNA array; and
(ii) extracting the unannealed RNA or cDNA molecules thereby generating processed RNA or cDNA;
and wherein the DNA array was produced by a method comprising:
(i) contacting a preparatory RNA sample with an oligonucleotide array, wherein the oligonucleotide array comprises two or more oligonucleotides linked to a surface, and wherein two or more RNA molecules from the preparatory RNA sample anneal to the oligonucleotides of the oligonucleotide array;
(ii) extending two or more of the oligonucleotides by reverse transcription using the annealed RNA molecules as templates to generate a DNA array comprising two or more DNA (cDNA) molecules;
(iii) disassociating the annealed RNA molecules from the DNA (cDNA) molecules; and
(iv) removing the preparatory RNA sample from the surface.

The test RNA sample and the preparatory RNA sample may be derived from soil sample(s), optionally the test RNA sample and the preparatory RNA sample are derived from the same soil sample. The methods defined herein may further comprise steps of providing a soil sample and extracting a preparatory RNA sample and a test RNA sample from the soil sample.

The method for processing cDNA may comprise:
(i) denaturing a first cDNA sample comprising double stranded cDNA molecules to produce single stranded cDNA molecules;
(ii) contacting the first cDNA sample with an oligonucleotide array, wherein the oligonucleotide array comprises two or more oligonucleotides linked to a surface, and wherein two or more cDNA molecules from the first cDNA sample anneal to the oligonucleotides of the oligonucleotide array;
(iii) extending two or more of the oligonucleotides using the annealed cDNA molecules as templates to generate a DNA array comprising two or more DNA molecules;
(iv) disassociating the annealed cDNA molecules from the DNA molecules of the DNA array;
(v) removing the first cDNA sample from the surface;
(vi) denaturing a second cDNA sample comprising double stranded cDNA molecules to produce single stranded cDNA molecules;
(vii) contacting the second cDNA sample with the DNA array, wherein one or more cDNA molecules from the second cDNA sample anneal to the DNA molecules;
(viii) extracting the unannealed cDNA molecules thereby generating processed cDNA.

By "array" is meant a collection or arrangement of oligonucleotide (DNA, optionally cDNA) molecules linked or attached to a (solid) surface. Multiple methods of linking oligonucleotides to a surface are available (for example amine-modified oligonucleotides covalently linked to an activated carboxylate group or succinimidyl ester, thiol-modified oligonucleotides covalently linked via an alkylating reagent such as an iodoacetamide or maleimide, Digoxigenin NHS Ester, cholesterol-TEG, biotin-modified oligonucleotides captured by immobilized streptavidin) and are well-known to the skilled person. The link may be covalent or non-covalent. The link may be direct or indirect. The DNA array may be a cDNA array.

The method for processing RNA may reduce the variability in the levels of the RNA (e.g. by at least 10%, at least 20%, at least 30%, at least 40%, at least 50%, at least 60%, at least 70%, at least 80%, or at least 90%). The method for processing RNA may achieve a more uniform distribution of RNA sequences. The difference in abundance between the most abundant RNA and the least abundant RNA may be reduced (e.g. by at least 10%, at least 20%, at least 30%, at least 40%, at least 50%, at least 60%, at least 70%, at least 80%, or at least 90%). The method for processing RNA may reduce the number of molecules (copy number) of the (1, 10, 100, 1000, or 10000) most abundant RNA molecule(s) by at least 10%, at least 20%, at least 30%, at least 40%, at least 50%, at least 60%, at least 70%, at least 80%, or at least 90%. The number of molecules (copy number) of the most abundant RNA molecule in the (second) RNA sample may be reduced by at least 50% in the processed RNA. The relative abundance of the (1, 10, 100, 1000, or 10000) least abundant RNA molecule(s) may be increased by at least 10%, at least 20%, at least 30%, at least 40%, at least 50%, at least 60%, at least 70%, at least 80%, or at least 90%. Thus, the method for processing RNA may be a method for normalizing RNA.

The method for processing cDNA may reduce the variability in the levels of the cDNA (e.g. by at least 10%, at least 20%, at least 30%, at least 40%, at least 50%, at least 60%, at least 70%, at least 80%, or at least 90%). The method for processing cDNA may achieve a more uniform distribution of cDNA sequences. The difference in abundance between the most abundant cDNA and the least abundant cDNA may be reduced (e.g. by at least 10%, at least 20%, at least 30%, at least 40%, at least 50%, at least 60%, at least 70%, at least 80%, or at least 90%). The method for processing cDNA may reduce the number of molecules (copy number) of the (1, 10, 100, 1000, or 10000) most abundant cDNA molecule(s) by at least 10%, at least 20%, at least 30%, at least 40%, at least 50%, at least 60%, at least 70%, at least 80%, or at least 90%. The number of molecules (copy number) of the most abundant cDNA molecule in the (second) cDNA sample may be reduced by at least 50% in the processed cDNA. The relative abundance of the (1, 10, 100, 1000, or 10000) least abundant cDNA molecule(s) may be increased by at least 10%, at least 20%, at least 30%, at least 40%, at least 50%, at least 60%, at least 70%, at least 80%, or at least 90%. Thus, the method for processing cDNA may be a method for normalizing cDNA.

The method for processing nucleic acid may reduce the variability in the levels of the nucleic acid (e.g. by at least 10%, at least 20%, at least 30%, at least 40%, at least 50%, at least 60%, at least 70%, at least 80%, or at least 90%). The method for processing nucleic acid may achieve a more uniform distribution of nucleic acid sequences. The difference in abundance between the most abundant nucleic acid and the least abundant nucleic acid may be reduced (e.g. by at least 10%, at least 20%, at least 30%, at least 40%, at least 50%, at least 60%, at least 70%, at least 80%, or at least 90%). the method for processing nucleic acid may reduce the number of molecules (copy number) of the (1, 10, 100, 1000, or 10000) most abundant nucleic acid molecule(s) by at least 10%, at least 20%, at least 30%, at least 40%, at least 50%, at least 60%, at least 70%, at least 80%, or at least 90%. The number of molecules (copy number) of the most abundant nucleic acid molecule in the (second) nucleic acid sample may be reduced by at least 50% in the processed nucleic acid. The relative abundance of the (1, 10, 100, 1000, or 10000) least abundant nucleic acid molecule(s) may be increased by at least 10%, at least 20%, at least 30%, at least 40%, at least 50%, at least 60%, at least 70%, at least 80%, or at least 90%. Thus, the method for processing nucleic acid may be a method for normalizing nucleic acid.

In theory, the maximum non-targeted sampling efficiency is produced if all unique nucleic acid sequences are represented at the same relative abundance. Thus the objective of normalization is to re-distribute a nucleic acid sample to meet this criterion as closely as possible.

Processed RNA, DNA or nucleic acid may be RNA, DNA or nucleic acid that is more readily analysable. It may be more efficiently sequenced because the relative representation of less abundant sequences is increased. Thus, the processed RNA, DNA or nucleic acid may be normalized RNA, DNA or nucleic acid, respectively.

Processed RNA may comprise RNA sequences having substantially the same levels. For example, wherein the levels of the sequences of the processed RNA vary by less than 50%, less than 40%, less than 30%, less than 20%, or less than 10%. The processed RNA may be a processed RNA sample in which at least a portion of the (1, 10, 100, 1000, or 10000) most abundant sequence(s) in the second RNA sample have been removed.

Processed cDNA may comprise cDNA sequences having substantially the same levels. For example, wherein the levels of the sequences of the processed cDNA vary by less than 50%, less than 40%, less than 30%, less than 20%, or less than 10%. The processed cDNA may be a processed cDNA sample in which at least a portion of the (1, 10, 100, 1000, or 10000) most abundant sequence(s) in the second cDNA sample have been removed.

Processed nucleic acid may comprise nucleic acid sequences having substantially the same levels. For example, wherein the levels of the sequences of the processed nucleic acid vary by less than 50%, less than 40%, less than 30%, less than 20%, or less than 10%. The processed nucleic acid may be a processed nucleic acid sample in which at least a portion of the (1, 10, 100, 1000, or 10000) most abundant sequence(s) in the second nucleic acid sample have been removed.

The invention provides a method for preparing normalized RNA comprising:
(i) providing a first RNA sample;
(ii) bringing the first RNA sample into contact with a surface, wherein the surface comprises one or more oligonucleotides, and wherein one or more RNA molecules from the first RNA sample anneal with the one or more oligonucleotides;
(iii) reverse transcribing one or more RNA molecules using one or more of the oligonucleotides as a primer to generate one or more cDNA molecules;
(iv) disassociating the one or more RNA molecules from the one or more cDNA molecules;
(v) removing the first RNA sample;
(vi) providing a second RNA sample;
(vii) bringing the second RNA sample into contact with the surface, wherein one or more RNA molecules from the second RNA sample anneal with the one or more cDNA molecules; and
(viii) extracting the unannealed RNA molecules for use as a normalized RNA sample.

The invention provides a method for preparing normalized cDNA comprising
(i) providing a first cDNA sample comprising double stranded cDNA molecules;
(ii) denaturing the first cDNA sample to produce single stranded cDNA molecules;
(iii) bringing the first cDNA sample into contact with a surface, wherein the surface comprises one or more oligonucleotides, and wherein one or more cDNA molecules from the first cDNA sample anneal with the one or more oligonucleotides;
(iv) synthesising one or more probe DNA molecules using the one or more oligonucleotides as a primer and the one or more cDNA molecules as a template;
(v) disassociating the one or more cDNA molecules from the one or more probe DNA molecules;
(vi) removing the first cDNA sample;
(vii) providing a second cDNA sample comprising double stranded cDNA molecules;
(viii) denaturing the second cDNA sample to produce single stranded cDNA molecules;
(ix) bringing the second cDNA sample into contact with the surface, wherein one or more cDNA molecules from the second cDNA sample anneal with the one or more probe DNA molecules;
(x) extracting the unannealed cDNA molecules for use as a normalized cDNA sample.

Normalizing a nucleic acid sample results in production of a normalized nucleic acid sample. By "normalized" is meant that the levels of RNA or cDNA sequences in the sample are more equal. To achieve this the relative representation or levels of less abundant sequences may be increased and/or the relative representation or levels of more abundant sequences may be decreased. Normalized RNA or cDNA may comprise RNA or cDNA sequences having substantially the same levels. For example, wherein the levels of the sequences of the normalized RNA or DNA vary by less than 50%, less than 40%, less than 30%, less than 20%, or less than 10%. The normalized RNA or cDNA may be a normalized RNA or cDNA sample in which at least a portion of the 10, 100, 1000, or 10000 most abundant sequences in the second RNA or cDNA sample have been removed. The methods for processing nucleic acid described herein may be methods for equalizing nucleic acid samples.

The methods of the invention may be employed with both RNA and DNA. However, the use of double stranded cDNA may require a denaturation step to produce single stranded DNA molecules. A strand selection may also be employed as part of the processing of double stranded cDNA. Oligo-dT molecules will only bind to the cDNA strand comprising the poly (A) sequence.

The method for processing cDNA may further comprise following the last step (step (viii)):
(a) contacting the unannealed cDNA molecules with a second oligonucleotide array wherein the second oligonucleotide array comprises two or more oligo-dT molecules linked to a surface, and wherein one or more of the cDNA molecules anneal with the oligo-dT molecules;
(b) removing the unannealed cDNA molecules from the surface; and
(c) disassociating the annealed cDNA molecules from the oligo-dT molecules for use as a processed cDNA sample.

The oligonucleotide(s) may be DNA molecules. The oligonucleotide(s) may comprise oligo-dT sequences (optionally 2 to 200, 5 to 200, 2 to 100, 5 to 50, 7 to 25 or 12 to 18 nucleotides long). The oligonucleotide(s) may be oligo-dT molecule(s). By oligo-dT molecule is meant a molecule comprising a stretch of deoxythymidine. The oligo-dT molecule may be of any length appropriate to bind to the poly(A) tail (a sequence of adenine nucleotides) of messenger RNA or the second strand of a double stranded cDNA molecule. The oligo-dT molecule(s) may be 2 to 100, 5 to 50, 7 to 25 or 12 to 18 nucleotides long. The oligo-dT molecule(s) may be at least 2, at least 5, at least 7, at least 12, at least 18 or at least 25 nucleotides long.

The oligonucleotide(s) may be immobilized on the surface. The surface may be two-dimensional such as a glass slides or three-dimensional such as micro-beads or microspheres. The surface may be one or more beads or spheres, optionally magnetic beads. The methods of the invention may also be carried out in a microfluidic flowcell.

The RNA (the first RNA sample and/or the second RNA sample) may comprise full length RNA.

The surface may comprise two or more oligonucleotides and the oligonucleotides may be optimally spaced so that the DNA molecules they prime do not interact with each other. The oligonucleotides may be optimally spaced so that the DNA (cDNA) molecules of the DNA array do not interact with each other. The optimal spacing for a given sample type may be determined based on the length of the DNA (cDNA) molecule expected to be produced. This is in turn determined by the (maximum) length of the RNA molecules in the first RNA sample or biological sample or cDNA molecules in the first cDNA sample or nucleic acid molecules in the (first) nucleic acid sample. The spacing between the oligonucleotides may be at least 1, at least 1.1, at least 1.2, at least 1.3, at least 1.4, at least 1.5, at least 1.6, at least 1.7, at least 1.8, at least 1.9, at least 2, at least 2.5, at least 3, at least 4 or at least 5 times the (maximum) length of the RNA molecules in the first RNA sample or biological sample or cDNA molecules in the first cDNA sample or nucleic acid molecules in the (first) nucleic acid sample. The spacing between the oligonucleotides may be between 1 and 5, between 1.3 and 3.5, between 1.4 and 3, or between 1.5 and 2.5 times the (maximum) length of the RNA molecules in the first RNA sample or biological sample or cDNA molecules in the first cDNA sample or nucleic acid molecules in the (first) nucleic acid sample. The spacing between the oligonucleotides may be 2 times the (maximum) length of the RNA molecules in the first RNA sample or biological sample or cDNA molecules in the first cDNA sample or nucleic acid molecules in the (first) nucleic acid sample. The spacing between the oligonucleotides may be at least 2 times the (maximum) length of the RNA molecules in the first RNA sample.

The oligonucleotides may be optimally spaced if the density of oligonucleotides (of the oligonucleotide array) is between 0.01 oligonucleotides per 1 micrometer squared and 10000 oligonucleotides per 1 micrometer squared, preferably between 0.1 oligonucleotides per 1 micrometer squared and 1000 oligonucleotides per 1 micrometer squared, more preferably between 1 oligonucleotide per 1 micrometer squared and 100 oligonucleotides per micrometer squared.

The first/preparatory RNA sample and the second/test RNA sample may be derived from the same (biological) sample. Likewise, the first cDNA sample and the second cDNA sample may be derived from the same (biological) sample. The first nucleic acid sample and the second nucleic acid sample may be derived from the same (biological) sample. Thus, from a given sample, for example a blood sample (optionally processed to extract RNA), a portion may be removed to form the first RNA sample and a further portion removed to form the second RNA sample. Likewise, from a given sample, for example a blood sample (optionally processed to generate cDNA), a portion may be removed to form the first cDNA sample and a further portion removed to form the second cDNA sample. Further, from a given sample, for example a blood sample (optionally processed to extract nucleic acid), a portion may be removed to form the first nucleic acid sample and a further portion removed to form the second nucleic acid sample. The first RNA sample (or first cDNA sample or first nucleic acid sample) and the second RNA sample (or second cDNA sample or second nucleic acid sample) may be derived from the same species, organism, tissue and/or cell type. The first RNA sample (or first cDNA sample or first nucleic acid sample) and the second RNA sample (second cDNA sample or second nucleic acid sample) may be derived from blood.

The method may further comprise sequencing the processed RNA, cDNA or nucleic acid. The method for processing nucleic acid (cDNA, RNA) may be a method for preparing nucleic acid (cDNA, RNA) for sequencing. Sequencing may be RNA or DNA sequencing. RNA may bereverse transcribed to cDNA prior to sequencing. Sequencing may detect and/or quantify the (target) nucleic acid molecules. Such methods comprise processing according to the invention followed by sequencing of the processed products, optionally using a next generation sequencing (NGS) platform. Examples of NGS platforms include Illumina sequencing (such as Hi-Seq and Mi-Seq), SMRT sequencing (Pacific Biosciences), Nanopore sequencing, SoLID sequencing, pyrosequencing (e.g. Roche 454) and Ion-Torrent (Thermo Fisher) which are well-known to the skilled person.

The invention is also concerned with RNA extraction.

The invention provides a method comprising:
(a) contacting a biological sample with an oligonucleotide array, wherein the oligonucleotide array comprises two or more oligonucleotides linked to a surface, wherein one or more RNA molecules from the biological sample anneal to the oligonucleotides of the oligonucleotide array;
(b) removing the unannealed sample from the surface; and
(c) disassociating the annealed RNA molecule(s) from the oligonucleotides to obtain an RNA sample.

The invention provides a method comprising:
(a) bringing a biological sample into contact with a surface, wherein the surface comprises one or more oligonucleotides, wherein one or more RNA molecules from the biological sample anneal with the one or more oligonucleotides;
(b) removing the unannealed sample; and
(c) disassociating the one or more RNA molecules from the one or more oligonucleotides to obtain an RNA sample.

These methods may be combined with other methods of the invention to provide an RNA sample. These methods may take place prior to step (i) of the methods recited herein. A portion of the obtained RNA sample may form the first RNA sample and a further portion may form the second RNA sample. The RNA sample may be reverse transcribed to cDNA. The oligonucleotide(s) may comprise one or more oligo-dT molecules. The oligonucleotide(s) may be oligo-dT molecules. Oligo-dT molecules will anneal with mRNA molecules with a poly(A) tail. The oligonucleotide(s) may comprise random or unique sequences to capture a range of RNAs in addition to mRNA. Custom oligonucleotide(s) may be designed to capture specific target RNA molecules (with complementary sequences). RNA molecules may be polyadenylated following extraction if they do not comprise a poly(A) tail.

After the processed RNA or cDNA is extracted, the method may further comprise disassociating the annealed RNA molecules from the cDNA molecules (or the annealed cDNA molecules from the DNA molecules). The disassociated molecules may be removed (optionally disposed of) leaving a surface comprising the cDNA molecules (or the DNA molecules). A further RNA or cDNA sample may then be processed using the surfaceThe method for processing RNA may further comprise, following step (vi) disassociating the annealed RNA molecules from the cDNA molecules and removing the disassociated RNA molecules from the surface and, optionally, repeating steps (v) and (vi) with a further RNA sample. The method for processing cDNA may further comprise, following step (viii) disassociating the annealed cDNA molecules from the DNA molecules and removing the disassociated cDNA molecules from the surface and, optionally, repeating steps (vi), (vii) and (viii) with a further cDNA sample.

The oligonucleotide(s) may be at least 5 nucleotides, at least 10 nucleotides, at least 100 nucleotides, at least 200 nucleotides or at least 500 nucleotides in length. The oligonucleotide(s) may consist of 5 to 200 nucleotides. The oligonucleotide array or surface may comprise at least 10, at least 100, at least 1000, at least 10000, at least 100000 or at least 1 million oligonucleotides. The oligonucleotide array or surface may comprise at least 1.1, at least 1.2, at least, 1.3, at least 1.4, at least 1.5, at least, 1.6, at least 1.7, at least 1.8, at least 1.9, at least 2, at least 3, at least 4, at least 5, at least 10, at least 100, or at least 1000 times as many oligonucleotides as there are RNA molecules in the first and/or second RNA sample, cDNA molecules in the first and/or second cDNA sample or nucleic acid molecules in the nucleic acid sample. The oligonucleotide array or surface may comprise at least 10, at least 100, at least 1000, at least 10000, at least 100000 or at least 1 million oligonucleotides with unique sequences (i.e. no two sequences are identical). The oligonucleotide(s) may comprise sequences complementary to the 10, 20, 50, 100, 1000 or 10000 most abundant RNAs (mRNAs) in a given sample, optionally the 10, 20, 50, 100, 1000 or 10000 most abundant RNAs (mRNAs) in human blood. The oligonucleotide(s) may comprise one or more sequences complementary to the mRNA coding for human serum albumin, one or more alpha globulins (for example haptoglobin), one or more beta globulins (for example plasminogen) and/or one or more gamma globulins.

The amount of RNA molecules in the (first and/or second) RNA sample or cDNA molecules in the (first and/or second) cDNA sample or nucleic acid molecules in the (first and/or second) nucleic acid sample may not not exceed the number of oligonucleotides in the oligonucleotide array and/or DNA molecules in the DNA array. The amount of RNA molecules in the second RNA sample may not exceed the number of cDNA molecules in the DNA array.

Biological sample and sample are used interchangeably herein. The (biological) sample may comprise a biological fluid or a fluid or lysate generated from a biological material. The biological fluid may comprise blood. Blood may be processed on the same day as collection, no more than 72 hours after collection, no more than 2 weeks after collection, no more than 4 weeks after collection or 4-12 months after collection. Blood may be stored at -80°C prior to processing. Plasma, and also serum, samples are envisaged. The sample may be a human sample. Sample types include other biological fluids such as saliva, urine or lymph fluid. Other sample types include solid tissues, including frozen tissue or formalin fixed, paraffin embedded (FFPE) material. These samples may be processed to lyse cells.

The RNA may be messenger RNA (mRNA), transfer RNA (tRNA), ribosomal RNA (rRNA), long non-coding RNA (IncRNA), small interfering RNA (siRNA), small nucleolar RNA (snoRNA), piwi-interacting RNA (piRNA), tRNA-derived small RNA (tsRNA), small rDNA-derived RNA (srRNA), microRNA (miRNA), or viral RNA etc.

The invention provides a system or device for performing a method as described herein.

The RNA sample may be processed using an RNA processing device for producing processed RNA from a biological sample (e.g. blood), the device comprising:
(i) a first module to receive the biological sample, wherein the first module comprises:
   (a) two or more oligonucleotides capable of annealing to one or more RNA molecules in the biological sample;
   (b) a first waste outlet to remove unannealed sample;
   (c) a sample outlet though which a portion of the sample comprising the one or more RNA molecules is capable of flowing following disassociation from the oligonucleotides; and
(ii) a second module to receive the one or more RNA molecules from the first module, wherein the second module comprises:
   (a) two or more oligonucleotides capable of annealing to one or more RNA molecules in the sample;
   (b) a processed RNA outlet through which the processed RNA can be obtained;
   (c) a waste RNA outlet to remove one or more RNA molecules;
wherein the first module and the second module together define a flow path along which a sample is capable of flowing.

The oligonucleotides may comprise oligo-dT sequences (optionally 2 to 200, 5 to 200, 2 to 100, 5 to 50, 7 to 25 or 12 to 18 nucleotides long). The oligonucleotides of the first module and/or the oligonucleotides of the second module may be oligo-dT molecules. By oligo-dT molecule is meant a molecule comprising a stretch of deoxythymidine. The oligo-dT molecule may be of any length appropriate to bind to the poly(A) tail (a sequence of adenine nucleotides) of messenger RNA or the second strand of a double stranded cDNA molecule. The oligo-dT molecule(s) may be 2 to 100, 5 to 50, 7 to 25 or 12 to 18 nucleotides long.

The oligonucleotides may comprise random or unique sequences to capture a range of RNAs in addition to mRNA. Custom oligonucleotides may be designed to capture specific target RNA molecules (with complementary sequences).

The oligonucleotides (oligo-dT molecules) of the first module may be linked to a first surface and the oligonucleotides (oligo-dT molecules) of the second module may be linked to a second surface. The first module may further comprise a sample inlet through which the biological sample is capable of entering the first module. The first module may further comprise a first reagent inlet through which reagents are capable of entering the first module and/or the second module may further comprise a second reagent inlet through which reagents are capable of entering the second module. The RNA processing device may further comprise temperature control means for adjusting the temperature of the first module and/or the second module. The first module may comprise a flow cell and/or the second module may comprise a flow cell. The oligonucleotides may be optimally spaced. Optimal spacing is discussed above. The spacing between the oligonucleotides of the first module and/or the second module may be at least 2 times the (maximum) length of the RNA molecules in the biological sample. The oligonucleotides may be optimally spaced if the density of oligonucleotides (linked to the first and/or second surface) is between 0.01 oligonucleotides per 1 micrometer squared and 10000 oligonucleotides per 1 micrometer squared, preferably between 0.1 oligonucleotides per 1 micrometer squared and 1000 oligonucleotides per 1 micrometer squared, more preferably between 1 oligonucleotide per 1 micrometer squared and 100 oligonucleotides per 1 micrometer squared.

The RNA processing device may further comprise a third module to receive the processed RNA, wherein the third module may comprise reagents for preparing the processed RNA for sequencing. The RNA processing device may further comprise a fourth module to receive the RNA prepared for sequencing, wherein the fourth module comprises sequencing reagents.

The devices disclosed herein may further comprise means for sequencing the processed RNA or cDNA, for example a sequencing machine or sequencer.

The devices disclosed herein may further comprise means for uploading the sequencing output to a cloud server.

The invention provides use of an RNA processing device as described herein in a method of normalizing RNA.

The method for processing nucleic acid may be a method for removing nucleic acid from a sample. Thus, the method for processing RNA may be a method for removing (abundant) RNA from the second RNA sample. Likewise, the method for processing cDNA may be a method for removing (abundant) cDNA from the second cDNA sample.

Where the surface or oligonucleotide array comprises one or more oligonucleotides with a sequence that is complementary to a target nucleic acid of interest, the target nucleic acid can bind to the one or more oligonucleotides. In this manner the target nucleic acid may be removed from a sample. The target nucleic acid may also be subjected to further processing such as sequencing.

The method for processing nucleic acid (processing the RNA sample) may comprise contacting a nucleic acid sample with a surface, wherein the surface comprises one or more oligonucleotides complementary to a target nucleic acid wherein the one or more oligonucleotides is at least 100 nucleotides in length and wherein the target nucleic acid anneals to the one or more oligonucleotides.

The oligonucleotide(s) may be at least 200 nucleotides in length, optionally at least 500 nucleotides in length. The surface may comprise two or more oligonucleotides. The oligonucleotide(s) may be linked to the surface.

The oligonucleotide(s) complementary to a target nucleic acid may be complementary to the full length (or at least 70%, at least 80%, at least 90% of the full length) of the target nucleic acid.

The methods may also comprise use of an RNA processing device for producing processed RNA from a biological sample, the device comprising:
(i) a first module to receive the biological sample, wherein the first module comprises:
   (a) two or more oligo-dT molecules capable of annealing to one or more RNA molecules in the biological sample;
   (b) a first waste outlet to remove unannealed sample;
   (c) a sample outlet though which a portion of the sample comprising the one or more RNA molecules is capable of flowing following disassociation from the two or more oligo-dT molecules; and
(ii) a second module to receive the one or more RNA molecules from the first module, wherein the second module comprises:
   (a) one or more oligonucleotide molecules complementary to a target RNA in the sample;
   (b) an unannealed sample outlet to remove unannealed sample;
   (c) a target RNA outlet through which the target RNA can be obtained following disassociation from the one or more oligonucleotide molecules
wherein the first module and the second module together define a flow path along which a sample is capable of flowing.

The one or more oligonucleotides complementary to the target RNA in the sample may be at least 100 nucleotides in length, preferably at least 200 nucleotides in length, more preferably at least 500 nucleotides in length.

The target nucleic acid may be from an RNA virus. The target nucleic acid may be (transcribed from) a bacterial gene such as an antibiotic resistance gene. The target nucleic acid may be a biomarker for a disease.

The magnetic beads for use in the claimed methods may also be provided in the form of a kit. Thus in a related aspect the methods may comprise use of a kit for processing an RNA sample, the kit comprising:
(a) one or more magnetic beads, wherein two or more oligo-dT molecules are linked to the one or more magnetic beads;
(b) a hybridization buffer; and
(c) a reverse transcriptase.

Any suitable reverse transcriptase may be included in the kit. Suitable buffers are also well known and commercially available.

The invention provides use of a kit as described herein in a method of normalizing RNA.

The invention provides a kit for processing a DNA sample, the kit comprising:
(a) one or more magnetic beads, wherein two or more oligo-dT molecules are linked to the one or more magnetic beads;
(b) a hybridization buffer; and
(c) a DNA polymerase.

Examples of DNA polymerases include thermostable polymerases such as Taq or Pfu polymerase and the various derivatives of those enzymes. Suitable buffers are also well known and commercially available.

The invention provides use of a kit as described herein in a method of normalizing cDNA.

The methods may comprise use of a kit for detection of a target nucleic acid in a sample, the kit comprising:
(a) one or more magnetic beads comprising one or more oligonucleotides complementary to the target nucleic acid wherein the one or more oligonucleotides is at least 100 nucleotides in length; and
(b) a hybridization buffer.

The hybridization buffer may comprise HEPES 1M (pH = 7.5), NaCl 5M and H₂0.

The kits of the invention may further comprise one or more, up to all, of dinucleotide triphosphates (dNTPs), MgCl₂ and a buffer.

The oligonucleotide(s) may comprise sequences complementary to the 10, 20, 50, 100, 1000 or 10000 most abundant RNAs (mRNAs) in a given sample, optionally the 10, 20, 50, 100, 1000 or 10000 most abundant RNAs (mRNAs) in human blood. The oligonucleotide(s) may comprise one or more sequences complementary to the mRNA coding for human serum albumin, one or more alpha globulins (for example haptoglobin), one or more beta globulins (for example plasminogen) and/or one or more gamma globulins.

Methods of RNA extraction and processing may be combined and incorporated into pipelines for analysing biological samples.

The invention provides a method of analysing a biological sample from a subject, the method comprising:
(a) extracting RNA from the biological sample;
(b) preparing a processed RNA sample; and
(c) sequencing the processed RNA.

The RNA may be full length RNA. The biological sample may comprise a biological fluid or a fluid or lysate generated from a biological material. The biological sample may be a liquid biopsy. The biological sample may be a blood sample, optionally a human blood sample.

Preparing a processed RNA sample may comprise RNA normalization (reducing the variability in the levels of different RNA sequences in the sample). Thus, the processed RNA sample may be a normalized RNA sample. By "normalized" is meant that the levels of RNA sequences in the sample are more equal. To achieve this the relative representation or levels of less abundant sequences may be increased and/or the relative representation or levels of more abundant sequences may be decreased. A normalized RNA sample may comprise RNA sequences having substantially the same levels. For example, wherein the levels of the sequences of the normalized RNA sample vary by less than 50%, less than 40%, less than 30%, less than 20%, or less than 10%. The normalized RNA may be a normalized RNA sample in which at least a portion of the 10, 100, 1000, or 10000 most abundant sequences in the sample have been removed.

Preparing a processed RNA sample may comprise equalizing the RNA sample. Thus, in the processed RNA sample the relative abundance of all the unique RNA sequences may be more equal. For example, the levels of the unique sequences in the processed RNA sample may vary by less than 50%, less than 40%, less than 30%, less than 20%, or less than 10%.

Preparing a processed RNA sample may reduce the variability in the levels of the RNA (e.g. by at least 10%, at least 20%, at least 30%, at least 40%, at least 50%, at least 60%, at least 70%, at least 80%, or at least 90%). Preparing a processed RNA sample may achieve a more uniform distribution of RNA sequences. In the processed RNA sample the difference in abundance between the most abundant RNA and the least abundant RNA may be reduced (e.g. by at least 10%, at least 20%, at least 30%, at least 40%, at least 50%, at least 60%, at least 70%, at least 80%, or at least 90%). Preparing a processed RNA sample may reduce the number of molecules (copy number) of the (1, 10, 100, 1000, or 10000) most abundant RNA molecule(s) by at least 10%, at least 20%, at least 30%, at least 40%, at least 50%, at least 60%, at least 70%, at least 80%, or at least 90%. The number of molecules (copy number) of the most abundant RNA molecule in the RNA sample may be reduced by at least 50% in the processed RNA. The relative abundance of the (1, 10, 100, 1000, or 10000) least abundant RNA molecule(s) may be increased by at least 10%, at least 20%, at least 30%, at least 40%, at least 50%, at least 60%, at least 70%, at least 80%, or at least 90% in the processed RNA.

The processed RNA sample may be more readily analysable. It may be more efficiently sequenced because the relative representation of less abundant sequences is increased.

The method may comprise diagnosing a disease in the subject.

The invention provides a method for diagnosing a disease in a subject, the method comprising:
(a) extracting RNA from a biological sample from the subject;
(b) preparing a processed RNA sample; and
(c) sequencing the processed RNA.

By diagnosing is meant determining that a subject has the disease at the time of testing.

The methods may comprise predicting a disease or identifying an increased risk of developing a disease.

The invention provides a method for predicting a disease or identifying an increased risk of developing a disease in a subject, the method comprising:
(a) extracting RNA from a biological sample from the subject;
(b) preparing a processed RNA sample; and
(c) sequencing the processed RNA.

By predicting is meant making a determination that a subject who at the time of testing does not have a disease is at an increased risk of developing a disease. The increased risk may be a risk higher than the average risk for the population. The increased risk may be a risk above a pre-calculated threshold level. The threshold level may be the point above which the benefits of increased monitoring and/or prophylactic treatment outweigh the negatives of potentially unnecessary intervention. The increased risk may be a percentage lifetime risk of greater than 1.5%, greater than 2%, greater than 5%, greater than 10%, greater than 50% or greater than 75%.

The methods may comprise selecting a treatment for a subject having a disease, predicting the responsiveness of a subject with a disease to a therapeutic agent and/or determining the clinical prognosis of a subject with a disease.

Sequencing the processed RNA allows the presence or absence and/or level of one or more RNA molecules to be determined. The presence or absence of one or more RNA molecules in the processed RNA sample may be used to identify whether the subject has the disease. The level of one or more RNA molecules in the processed RNA sample may be used to identify whether the subject has the disease. A comparison with a reference point or value may be used to diagnose, or predict a clinical condition or outcome. While as few as one specific RNA molecule (an RNA molecule with a specific sequence) may be used to diagnose or predict a clinical prognosis or response to a therapeutic agent, the specificity and sensitivity or diagnosis or prediction accuracy may increase using more RNA molecules (of other specific sequences).
the RNA extracted from the sample may comprise cell-free RNA.

Extracting RNA from the biological sample may comprise:
(a) contacting the biological sample with an oligonucleotide array wherein the oligonucleotide array comprises two or more oligonucleotides linked to a surface and wherein one or more RNA molecules from the sample anneals to the oligonucleotides of the oligonucleotide array;
(b) removing the unannealed sample from the surface; and
(c) disassociating the annealed RNA molecules from the oligonucleotides thereby generating an RNA sample.

The oligonucleotides may comprise one or more oligo-dT sequences. The oligonucleotides may be oligo-dT molecules.

Extracting RNA from the biological sample produces extracted RNA. Preparing a processed RNA sample may comprise following the steps of the method(s) for processing RNA defined above. Preparing a processed RNA sample may comprise taking a portion of the extracted RNA formed by extracting RNA from the biological sample to be a first RNA sample and a portion of the extracted RNA to be a second RNA sample and following the steps of the method(s) for processing RNA defined above. Preparing a processed RNA sample may comprise taking a portion of the extracted RNA to be a first RNA sample and a portion of the extracted RNA to be a second RNA sample and:
(i) contacting the first RNA sample with an oligonucleotide array, wherein the oligonucleotide array comprises two or more oligonucleotides linked to a surface, and wherein two or more RNA molecules from the first RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the first RNA sample from the surface;
(v) contacting the second RNA sample with the DNA array, wherein one or more RNA molecules from the second RNA sample anneal to the cDNA molecules; and
(vi) extracting the unannealed RNA molecules thereby generating processed RNA.

Preparing a processed RNA sample may comprise:
(i) contacting the extracted RNA with an oligonucleotide array (e.g. a DNA, optionally a cDNA array), wherein one or more RNA molecules from the extracted RNA anneal to one or more oligonucleotides of the oligonucleotide array; and
(ii) extracting the unannealed RNA molecules thereby generating processed RNA.

The method of analysing a biological sample from a subject may comprise use of one or more of the RNA processing device(s) and kit(s) of the present invention.

In specific embodiments sequencing the processed RNA comprises long-read sequencing.

The invention provides use of a method, device or kit as described herein in a method for determining soil microfauna composition.

The invention provides use of a method for processing a (test) RNA or cDNA sample in a method for determining soil microfauna composition, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

Soil microfauna composition may be determined through the identification of microorganisms and/or viruses present in a soil sample. This may be achieved through the identification of sequences in the sequencing output.

The invention provides use of a method, device or kit as described herein in ecological research.

The invention provides use of a method for processing a (test) RNA or cDNA sample in ecological research, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

Ecological research may comprise sequencing processed RNA or cDNA from one or more species. Ecological research may comprise obtaining information regarding the transcriptome of one or more species. Ecological research may lead to improved understanding of the overall biology of one or more species and/or how one or more species impact their local ecology.

The invention provides use of a method, device or kit as described herein in a method for assessing water quality.

The invention provides use of a method for processing a (test) RNA or cDNA sample in a method for assessing water quality, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

Water quality may be assessed through the identification/detection of microorganisms (for example bacteria and/or fungi) and/or viruses. Water quality may be assessed by extracting RNA from a water sample from a water source. This may be achieved through the identification of sequences in the sequencing output.

The invention provides use of a method, device or kit as described herein in a method for screening for dangerous biological material.

The invention provides use of a method for processing a (test) RNA or cDNA sample in a method for screening for dangerous biological material, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

The dangerous biological material may comprise a fungus, bacterium and/or a virus, optionally a pathogenic fungus, bacterium or virus, and/or a fungal, plant or animal derived toxin or drug that may still contain traces of nucleic acid (for example RNA). The screening may take place at a travel gateway.

The invention provides use of a method, device or kit as described herein in a method for confirming the identity of a subject.

The invention provides use of a method for processing a (test) RNA or cDNA sample in a method for confirming the identity of a subject, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

Confirming the identify of a subject may be achieved through the identification of sequences in the sequencing output. Confirming the identity of a subject may comprise DNA fingerprinting or include steps from a DNA fingerprinting method. Confirming the identify of a subject may be achieved through analysis of repetitive sequences that are highly variable, for example variable number tandem repeats, optionally short tandem repeats. The method may take place at a travel gateway.

The invention provides use of a method, device or kit as described herein in a process of RNA or DNA sequencing, optionally for discovery of new RNA and/or detection of low abundance RNA, further optionally wherein the sequencing is single cell sequencing.

The invention provides use of a method, device or kit as described herein in a process of metagenomic sequencing for discovery of new microbes and/or detection of low abundance microbes.

The invention provides use of a method, device or kit as described herein in a process of screening DNA or RNA samples, or screening genetic samples for the presence of infectious diseases.

The invention provides use of a method, device or kit as described herein in a process of detecting a nucleic acid biomarker, optionally a disease biomarker, further optionally a cancer biomarker.

The method may further comprise reporting the result. The result may be in the form of an RNA or DNA sequence, an indication of the presence or absence of a microbe or disease and/or an indication of the presence or absence or level of a disease biomarker.

### DESCRIPTION OF THE FIGURES

The invention will now be described in further detail, by way of example only, with reference to the following examples and the accompanying figures.
**Figure 1** - A schematic representation of a magnetic bead with oligo-dT primers attached. During RNA processing according to the invention RNA molecules anneal to the oligo-dT molecules and reverse transcription creates a cDNA copy attached to the bead.
**Figure 2** - A schematic representation of a magnetic bead with cDNA probes attached. The magnetic bead is re-introduced to another batch of RNA. A portion of the RNA anneals to the probes (captured RNA). The beads are then immobilized and the solution extracted comprising the normalized RNA.
**Figure 3** - A schematic representation of a microfluidic flowcell in use for RNA processing according to the invention. RNA flows through and the temperature is cooled down to allow for poly-A annealing to the oligo-dT forest.
**Figure 4** - A schematic representation of a microfluidic flowcell in use for RNA processing according to the invention. Reverse transcription materials are added and incubation for reverse transcription carried out.
**Figure 5** **-** A schematic representation of a microfluidic flowcell in use for RNA processing according to the invention. Heating disassociates RNA which is then flushed out.
**Figure 6** **-** A schematic representation of a microfluidic flowcell in use for RNA processing according to the invention showing the cDNA forest ready for RNA to be normalized.
**Figure 7** **-** A schematic representation of a microfluidic flowcell in use for RNA processing according to the invention. New RNA is added and incubated between 45°C and 75°C (for example, at around 68°C) for association. The abundant RNA anneals to the cDNA forest while the free normalized RNA flows through.
**Figure 8** - A schematic representation of a microfluidic flowcell in use for RNA processing according to the invention. Heating to between 80°C and 100°C (for example, 98°C) disassociates RNA which flows through to waste. The cycle starting from Figure 7 can then be repeated.
**Figure 9** - A schematic representation of RNA extraction according to the invention. A sample with lysed cells flows over the surface. Cooling down the temperature allows for poly-A annealing to the oligo-dT forest. The flowcell is flushed leaving only bound RNA. Heating up disassociates RNA which flows through to the next step.
**Figure 10** - A schematic representation of an RNA processing device according to the invention.
**Figure 11** - A schematic representation of RNA processing according to the invention where the oligonucleotides linked to the surface are complementary to target RNA (designed probe cDNA forest). A sample with lysed cells flows through. Incubation between 45°C and 75°C (for example, at around 68°C) allows for full length association. The cell is flushed leaving only bound RNA. Heating up disassociates the RNA which flows though for further processing.
**Figure 12** - A schematic representation of DNA processing according to the invention where the oligonucleotides linked to the surface are complementary to target DNA (designed probe cDNA forest). A sample with lysed cells and fragmented DNA flows through. Heating to between 80°C and 100°C (for example, 98°C) disassociates double strands. Incubation between 45°C and 75°C (for example, at around 68°C) allows for full length association. The cell is flushed leaving only bound DNA. Heating up disassociates the DNA which flows though for further processing.

### DETAILED DESCRIPTION

The invention is based on methods that take advantage of the ability to generate full length sequences from RNA extracted from blood without fragmenting RNA or cDNA products before sequencing. This provides a transcriptome representing any RNA that make its way into the circulatory system including RNA from typical blood cells (like red blood cells, white blood cells, other immune cells, etc.), RNA from any other cells that are typically uncommon in the circulatory system such as cancer cells or cells that somehow dislodged into the circulatory system and extracellular RNA which could have originated from any cell within the body. By detecting RNA from all these sources and at full length the present inventors can ascribe each RNA to a cell/tissue of origin as well as a state of cell or tissue behaviour. The transcription start site, end site, and splicing are features which typically represent unique combinations used by different cell types. This information can also be used to directly identify the protein isoform that would be translated from messenger RNA. The ability to detect low level signals of full length RNA which can then be translated into protein isoforms means the present inventors can detect unique protein isoforms that are expressed exclusively by certain cells, for example cancer cells. These isoforms will have sections of their peptide sequence which are unique to the specific cells, for example the cancer cells. These cancer specific peptide sub-sequences are also known as neo-antigens because they are typically presented on the cell surface via MHC complexes.

Detecting isoform based neo-antigens, as opposed to mutation based neo-antigens, has particular advantages. For example, it means that instead of having to design a new vaccine for each patient based on the unique mutation presenting in each patient, the present methods make it possible to identify a set of isoform based neo-antigens that are represented across a large percentage of the population. This means that RNA cancer vaccines can be based on that set instead of having to design new vaccines for each patient. With this paradigm, the development, safety testing, production, QC, and delivery of RNA cancer vaccines can be managed much more economically and with less risk of negative effects. This means that RNA cancer vaccines can be more affordable and also means that RNA cancer vaccines could be used more often since they would be safer and more cost effective.

The use of blood samples rather than tumour samples to find the neoantigen targets is also advantageous. With tumour samples there is a limited amount of material that may be difficult to obtain and only available at a particular time point. This also means targets are based on the removed primary cells when the aim is to target the remaining distal cells that are likely to be genetically different. If targets are not identified that span all remaining cancer cells it creates selective pressure to allow cancer cells that do not present the targets to thrive and cause recurrence. This may even encourage the development of cancers with higher mutations rates that could be more problematic.

As the present methods allow for continuous monitoring of patients for neo-antigen targets and because they could help make RNA cancer vaccines cheaper and safer, RNA cancer vaccine therapy could be used early and often. It can be applied multiple times depending on the neo-antigen readouts obtained from the present methods until it is observed that there are no longer any neo-antigens presenting in the blood of the patient.

Neo-antigen targets also represent unique structures in cancer proteins that could be exploited for new drug development. Since a large percentage of targeted cancer therapies interact with proteins, data obtained from the present methods could be used to inform usefulness of a wide range of cancer therapies, including check point inhibitors.

The present methods can detect diseases at the earliest stages and indicate best therapies all from one testing paradigm. This allows for not only earlier treatment of diseases thus increasing success rates but also reducing time to treatment after diagnosis. The process could also be applied to prevent the formation of tumours or damaging cancers by screening the population and applying RNA cancer vaccines before cancer cells have the chance to grow to any meaningful size.

The data collected using the present methods can be used to compile an extensive full length RNA human database which can be used to data mine new potential drug targets.

In summary, unlike with mutation based methods which have to be designed for each patient, the present methods use isoform level targets which should be common across patients. This means it is possible to identify a set of targets that would work for a large percentage of the population. So RNA cancer vaccines can be developed, tested, and produced for each of the targets in the set of targets and applied individually or in combination when detected using the methods herein. This vastly reduces the cost and increases the safety which means RNA cancer vaccines could be used early and often. This means that that it is not necessary to destroy all the cancer cells in one go. The present methods allow for monitoring and adjusting treatment until all neoantigens in the blood disappear. The present methods could also guide complementary treatments like checkpoint inhibitors so that combination therapies could be applied when needed. Thus, the present methods could be applied to both prevent cancer formation and treat already formed cancer.

These concepts could be applied in a very similar way with a wide range of diseases including autoimmune, diabetes, coronary, metabolic, and others.

### RNA/cDNA processing

RNA or cDNA samples are typically dominated by sequences from highly expressed genes. Normalization to achieve a more uniform distribution of sequences can increase the efficiency of sequencing for transcript discovery and/or detection. First, genes and isoforms which are specific to the condition in question are easier to detect, and second, there is less redundancy in data generated reducing data storage requirements.

Thus, the methods provided herein may include a processing step in the form of RNA or cDNA normalization.

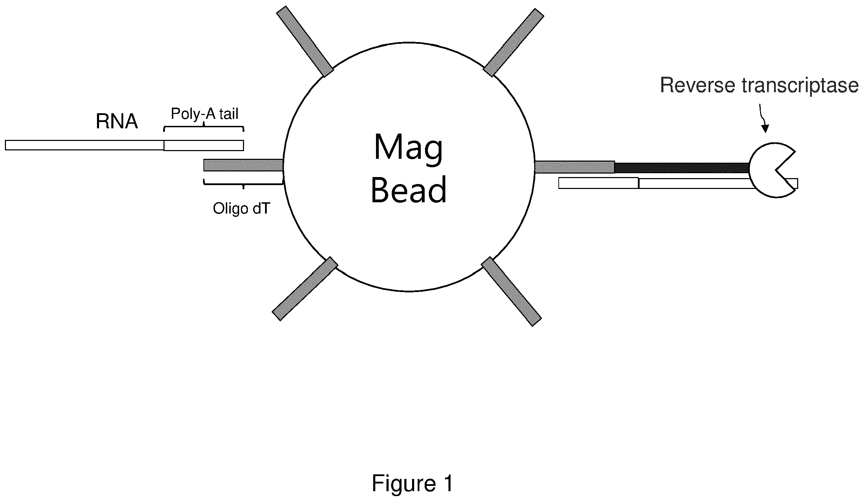

Figure 1 shows schematically an array of oligonucleotides, in this case oligo-dT molecules linked to a magnetic bead. A first RNA sample is contacted with the magnetic bead and RNA molecules comprising a poly-A tail anneal to the oligo-dT molecules. The oligo-dT molecules are extended by reverse transcription using the annealed RNA molecules as templates to generate cDNA molecules linked to the bead (a DNA array). Abundant RNA molecules (RNA sequences that occur more frequently in the sample) will produce more cDNA molecules. The annealed RNA molecules are disassociated from the cDNA molecules and the first RNA sample removed from the magnetic bead leaving the cDNA molecules linked to the bead.

This stage of the method to generate the cDNA molecules linked to the bead (DNA array) involves the following steps:
(i) Add magnetic beads with oligo-dT molecules to purified RNA solution (first RNA sample);
(ii) Heat up to between 65°C and 100°C (above 70°C, optionally between 70°C and 100°C, between 80°C and 100°C or between 90°C and 100°C) for 5 second to 1 minute to remove secondary structures;
(iii) Cool down to below 65°C (i.e. 65°C or below, for example between 30°C and 65°C) for 5 seconds to 1 minute to anneal oligo-dT molecules to poly-A tails of RNA;
(iv) Add reverse transcription materials (reverse transcriptase, dNTP, etc.);
(v) Incubate for reverse transcription (at between 30°C and 80°C, optionally at between 30°C and 60°C, for 1 minute to 2 hours);
(vi) Heat up to between 80°C and 100°C (for example, around 98°C) for 5 seconds to 1 minute to disassociate RNA from cDNA copy;
(vii) Immobilize beads using external magnet;
(viii) Remove liquid solution containing RNA.

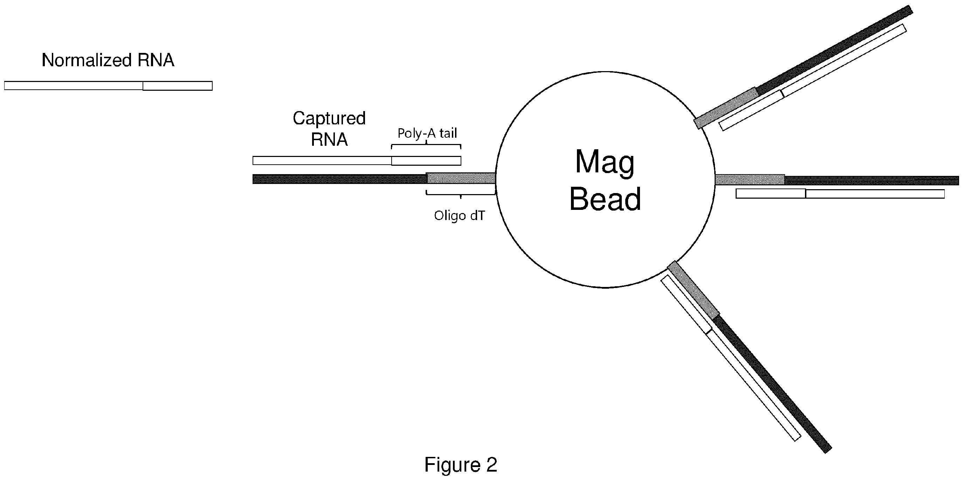

A second RNA sample is then contacted with the bead comprising the linked cDNA molecules. As shown in Figure 2, RNA molecules from the second RNA sample anneal to the cDNA molecules with the complementary sequence. As abundant RNA molecules produce more cDNA molecules in the stage shown in Figure 1, more of the abundant RNA molecules in the second RNA sample will be captured by the cDNA molecules then will be the case for the less abundant RNA molecules. The RNA molecules that do not anneal to the cDNA molecules, therefore, have a more uniform distribution of sequences - the RNA is normalized as it is no longer dominated by a few very abundant sequences. The magnetic bead is then immobilized and the unannealed RNA molecules are extracted thereby generating processed RNA.

The amount of RNA molecules in the second RNA sample should ideally not exceed the number of DNA molecules in the DNA array (cDNA forest) for each reaction cycle. If the DNA array outnumbers each pass of RNA it ensures there are enough probes to anneal to the high abundance RNA.

This stage of the method to generate the processed RNA involves the following steps:
(i) Add new aliquot of RNA solution (second RNA sample), optionally from the same sample;
(ii) Heat up to between 65°C and 100°C (above 70°C, optionally between 70°C and 100°C, between 80°C and 100°C or between 90°C and 100°C) for 5 seconds to 1 minute to remove secondary structures;
(iii) Cool down and incubate between 45°C and 75°C (for example, at around 68°C) for 10 second to 8 hours to allow for controlled full length association with cDNA;
(iv) Immobilize magnetic bead with external magnet;
(v) Extract liquid solution containing processed (normalized) RNA fraction;
(vi) Repeat with further aliquots of RNA solution to get more processed RNA (following disassociation of the annealed RNA).

The present invention makes possible the normalization of full length RNA. The advantages of analysing RNA directly include the fact that it is not necessary to do PCR (saves time and reagents and no PCR artefacts), lack of bias, nanopore sequencing can directly detect modifications present in RNA (modifications change the way in which RNA moves through pores).

The oligonucleotide array may be linked to any appropriate surface and the present invention is not limited to the use of magnetic beads. For example, the method may also be carried out in a microfluidic flowcell.

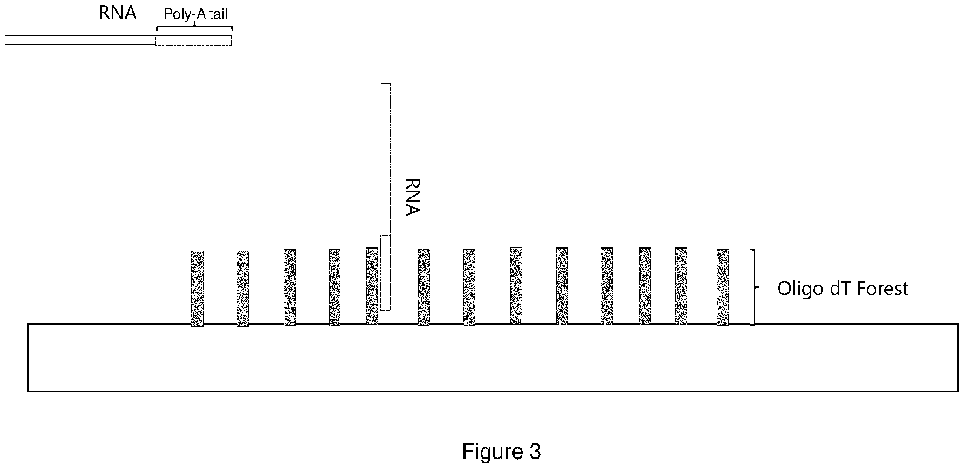

Figure 3 shows schematically an array of oligonucleotides, in this case oligo-dT molecules (also termed oligo-dT forest herein), linked to the surface of a flowcell. A first RNA sample flows through the flowcell and RNA molecules comprising a poly-A tail anneal to the oligo-dT molecules. The temperature is cooled down to below 65°C (i.e. 65°C or below, optionally between 30°C and 65°C) for the oligo-dT molecules to anneal to the poly-A tails of the RNA.

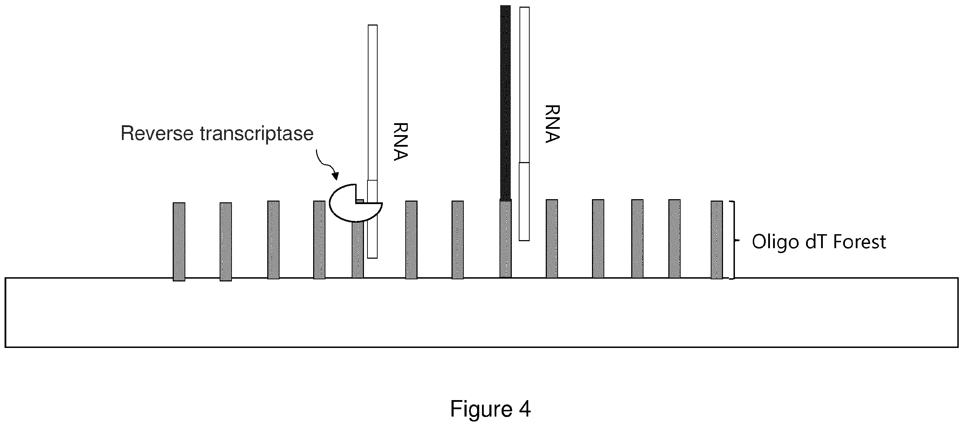

Reverse transcription reagents are added and incubation carried out. As shown in Figure 4 the oligo-dT molecules are extended by reverse transcription using the annealed RNA molecules as templates to generate cDNA molecules linked to the surface via the oligo-dT sequences (a DNA array). Abundant RNA molecules (RNA sequences that occur more frequently in the sample) will produce more cDNA molecules.

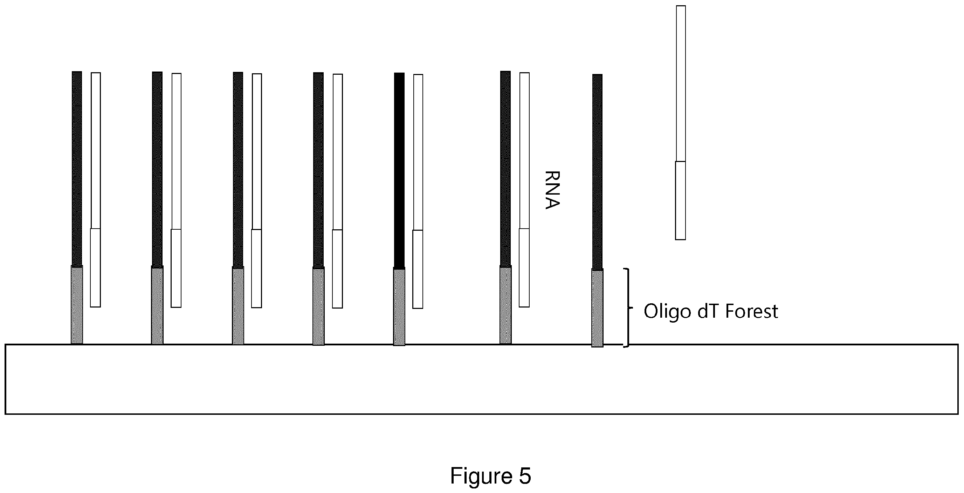

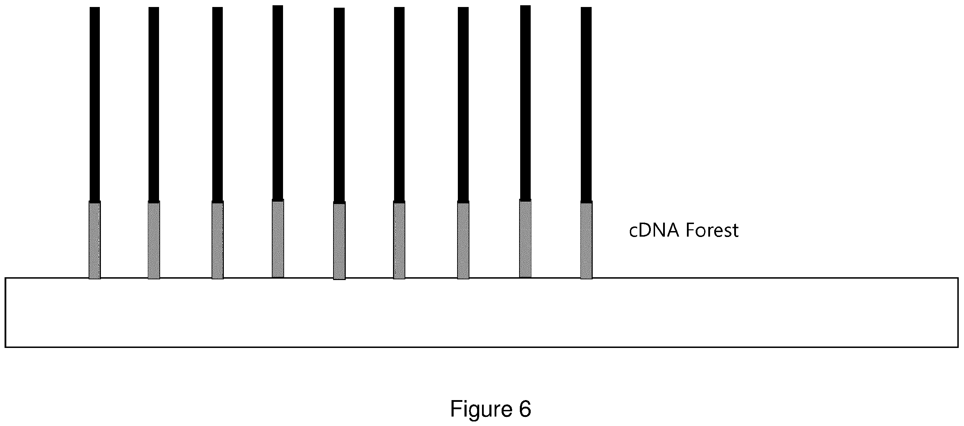

As shown in Figure 5 the annealed RNA molecules are disassociated from the cDNA molecules by heating. The first RNA sample is then flushed out leaving the cDNA molecules linked to the surface (DNA array or cDNA forest) as shown in Figure 6.

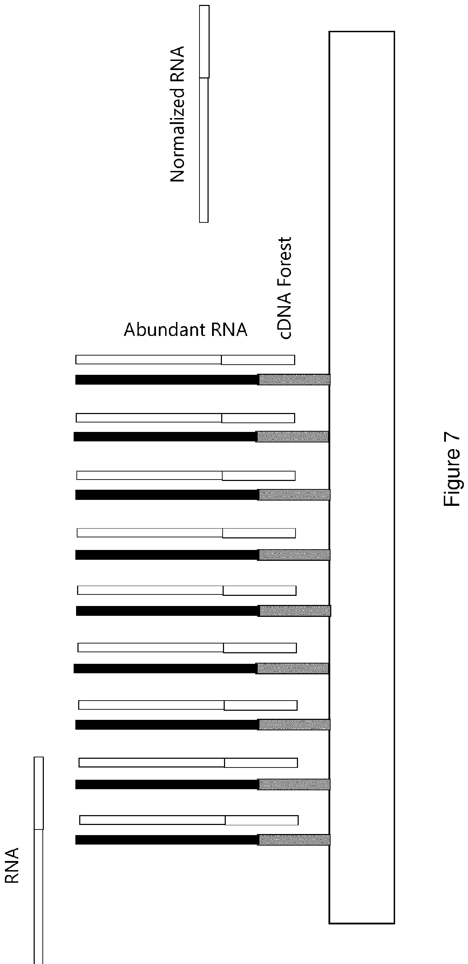

A second RNA sample is then contacted with the surface comprising the cDNA molecules and incubated at around 68°C. As shown in Figure 7, RNA molecules from the second RNA sample anneal to the cDNA molecules with the complementary sequence. As abundant RNA molecules produce more cDNA molecules in the step shown in Figure 4, more of the abundant RNA molecules in the second RNA sample will be captured by the cDNA molecules then will be the case for the less abundant RNA molecules. The RNA molecules that do not anneal to the cDNA molecules, therefore, have a more uniform distribution of sequences - the RNA is normalized as it is no longer dominated by a few very abundant sequences. The unannealed RNA molecules flow through thereby generating processed RNA.

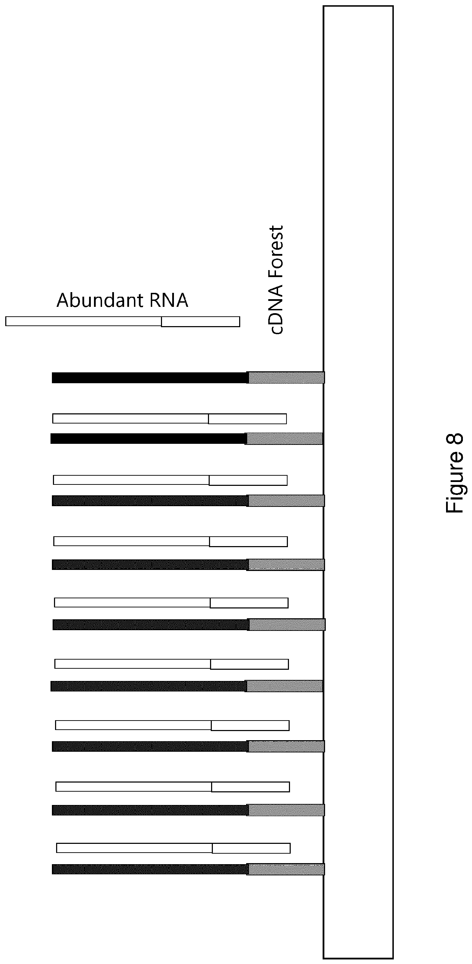

Figure 8 illustrates a further step of disassociating the annealed RNA molecules from the cDNA molecules by heating to 98°C. The disassociated RNA flows through to waste. The surface comprising the cDNA molecules can then be re-used with further RNA samples to generate more processed RNA.

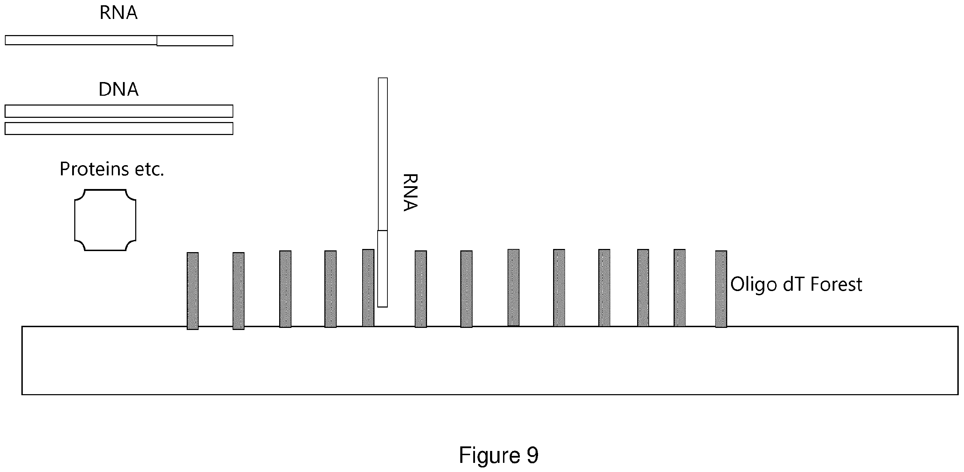

As illustrated in Figure 9 a similar principle can be applied to RNA extraction. A biological sample with lysed cells comprising RNA, DNA, proteins etc. flows over an array of oligonucleotides, in this case oligo-dT molecules (also termed oligo-dT forest herein) linked to the surface of a flowcell. The temperature is cooled down to below 65°C (i.e. 65°C or below, optionally between 30°C and 65°C) for the oligo-dT molecules to anneal to the poly-A tails of the RNA. The flowcell is flushed to leave only the annealed RNA. The temperature is then increased to between 80°C and 100°C (for example, 98°C) to disassociate the annealed RNA molecules from the oligonucleotides to obtain an RNA sample. The RNA then flows through for further processing.

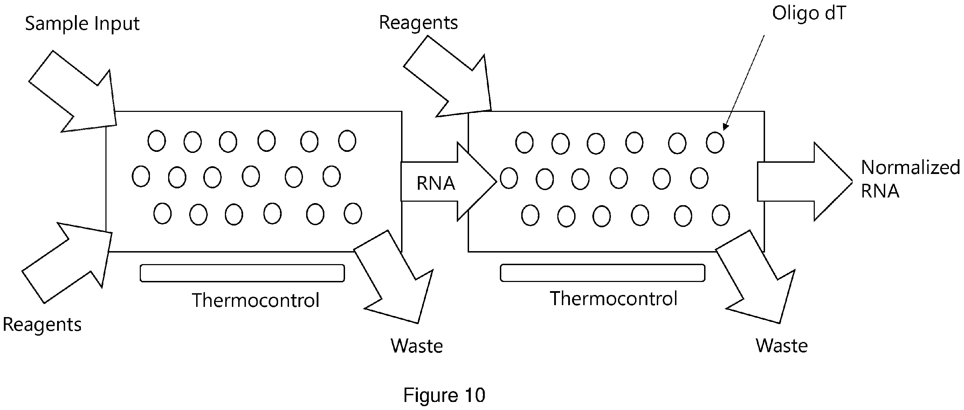

RNA extraction and RNA processing can be linked through combining microfluidic flowcells. One flowcell (also termed module or reaction chamber herein) extracts RNA which is then processed in a further flowcell (or module). An RNA processing device is illustrated schematically in Figure 10, which comprises two flowcells. The biological sample is input through a sample inlet in the first flowcell. The biological sample may be a sample of lysed cells comprising RNA, DNA, proteins etc. Reagents enter through a reagent inlet, for example buffer and/or RNA stabilising reagents. The surface of the first flowcell is as shown in Figure 9 i.e. an array of oligonucleotides, in this case oligo-dT molecules, linked to the surface of the flowcell. Both the first and second flowcells comprise temperature control means (thermocontrol) for adjusting the temperature. The temperature control means allow the temperature to be cooled down to below 65°C (i.e. 65°C or below, optionally between 30°C and 65°C) for the oligonucleotides to anneal to the RNA. The first flowcell comprises a first waste outlet to remove unannealed sample such that when the first flowcell is flushed only the annealed RNA is left. The temperature control means then allow the temperature to be increased to between 80°C and 100°C (for example, 98°C) to disassociate the annealed RNA molecules from the oligonucleotides to obtain an RNA sample. The first flow cell comprises a sample outlet though which RNA sample is capable of flowing following disassociation from the oligonucleotides. The first flowcell and the second flowcell together define a flow path along which the sample is capable of flowing. Thus, the first and second flowcells are joined by a connecter, for example a tube, that allows the RNA to flow from the first flowcell to the second flowcell for further processing.

The RNA sample enters through an RNA sample inlet in the second flowcell. The second flowcell (module) also comprises a second reagent inlet through which reagents are capable of entering the second flowcell. The surface of the second flowcell comprises an array of oligonucleotides, (for example oligo-dT molecules) linked to the surface of the flowcell. The RNA sample flows through the flowcell and RNA molecules anneal to the oligonucleotides. Reverse transcription reagents are added through the second reagent inlet. The oligonucleotides are extended by reverse transcription using the annealed RNA molecules as templates to generate cDNA molecules linked to the surface (a DNA array). The annealed RNA molecules are disassociated from the cDNA molecules by heating using the temperature control means. Thus, the temperature control means are capable of heating the RNA molecules to 98°C. The second flowcell comprises a waste RNA outlet to remove one or more RNA molecules. The waste RNA outlet allows the RNA sample to be flushed out leaving the cDNA molecules linked to the surface.

A further RNA sample then enters the second flowcell, contacts the surface comprising the linked cDNA molecules and is incubated between 45°C and 75°C (for example, at around 68°C). RNA molecules from the further RNA sample anneal to the cDNA molecules with the complementary sequence. The second flowcell comprises a processed RNA outlet through which the unannealed RNA molecules flow through thereby generating processed (normalized) RNA.

Where the surface or oligonucleotide array comprises one or more oligonucleotides with a sequence that is complementary to a target nucleic acid of interest, the target nucleic acid can bind to the one or more oligonucleotides. In this manner the target nucleic acid may be removed from a sample. The target nucleic acid may also be subjected to further processing such as sequencing. A further flowcell may be included in the RNA processing device discussed above that comprises one or more oligonucleotides with a sequence that is complementary to a target nucleic acid of interest. Alternatively the second flowcell may comprise one or more oligonucleotides with a sequence that is complementary to a target nucleic acid of interest.

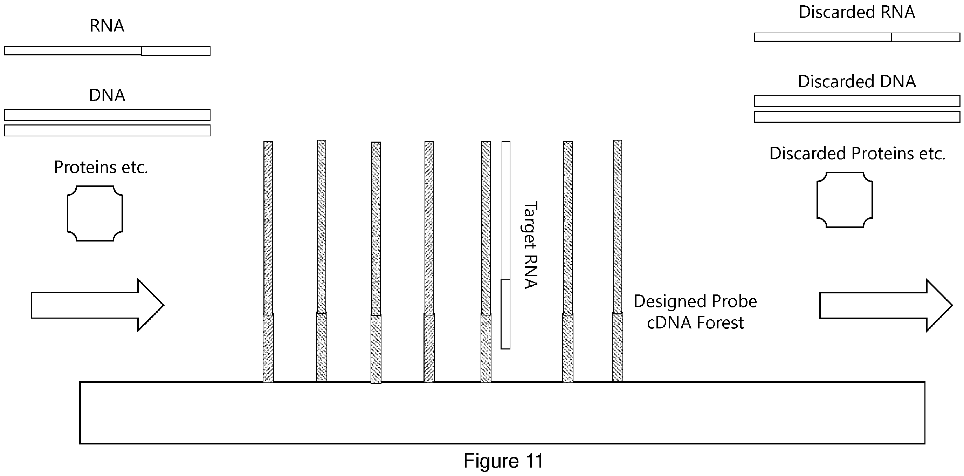

The target nucleic acid may also be directly extracted from a biological sample. Figure 11 shows a surface or array that comprises oligonucleotides complementary to a target RNA (also termed designed probe cDNA forest herein). The oligonucleotides are at least 100 nucleotides in length. A biological sample with lysed cells comprising RNA, DNA, proteins etc. flows over the array of oligonucleotides. Incubation between 45°C and 75°C (for example, at around 68°C) allows for full length association of target RNA with the oligonucleotides. The flowcell is flushed to leave only the annealed RNA. The temperature is then increased to between 80°C and 100°C (for example, 98°C) to disassociate the annealed RNA molecules from the oligonucleotides to obtain the target RNA. The RNA then flows through for further processing. The remaining RNA, DNA and protein may then be discarded or further processed.

The methods, devices and kits described herein (when used to process RNA) can be used to target RNA viruses, bacterial genes such as antibiotic resistance genes and RNA biomarkers for disease. Coupled with RNA sequencing this allows for precise diagnostics. The DNA array (probe forest) can be reused. Thus the device can be used as a quick reusable screening for viral infection if coupled with (Nanopore) sequencing or another detection method (PCR, LAMP, etc.). The methods, kits and devices can also be used in agritech to monitor crops and livestock for diseases. Sample processing is fast and efficient.

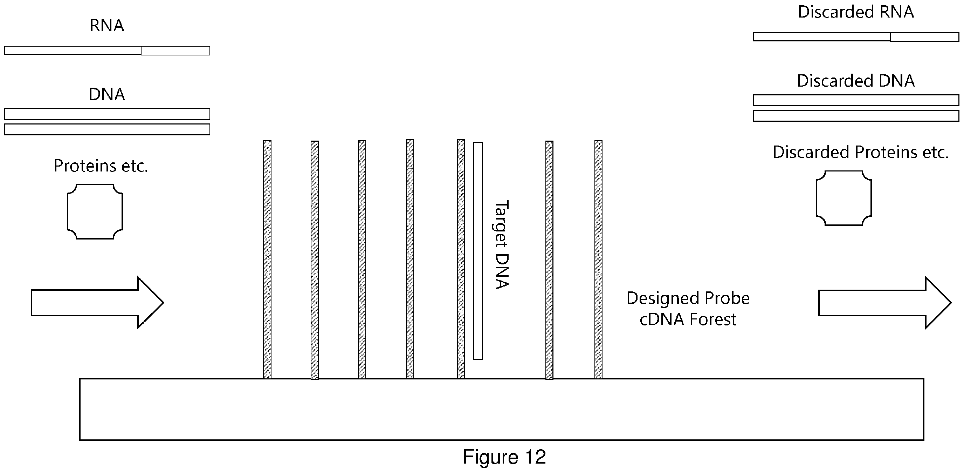

The methods, devices and kits described herein may also be used to process DNA. However, the use of double stranded DNA requires a denaturation step to produce single stranded DNA molecules. Figure 12 shows a surface or array that comprises oligonucleotides complementary to a target DNA (also termed designed probe cDNA forest herein). The oligonucleotides are at least 100 nucleotides in length. A biological sample with lysed cells comprising RNA, fragmented DNA, proteins etc. flows over the array of oligonucleotides. The sample is heated to between 80°C and 100°C (for example, 98°C) to disassociate the double stranded DNA. Then incubation between 30°C and 75°C (for example, at around 68°C) allows for full length association of target DNA with the oligonucleotides. The flowcell is flushed to leave only the annealed DNA. The temperature is then increased to between 80°C and 100°C (for example, 98°C) to disassociate the annealed DNA molecules from the oligonucleotides to obtain the target DNA. The target DNA then flows through for further processing. The remaining RNA, DNA and protein may then be discarded or further processed.

The methods, devices and kits described herein (when used to process DNA) can be used to target DNA viruses, for bacterial identification (and identification of other microbes), to detect DNA biomarkers for disease and for rapid DNA identification as a means of validating individual identities. The DNA array (probe forest) can be reused. Thus the device can be used as a quick reusable screening for viral infection if coupled with (Nanopore) sequencing or another detection method (PCR, LAMP, etc.). The methods, kits and devices can also be used in agritech to monitor crops and livestock for diseases. Sample processing is fast and efficient.

Where oligonucleotide(s) complementary to a target nucleic acid are employed they may be complementary to the full length (or at least 70%, at least 80%, or at least 90% of the full length) of the target nucleic acid. This differs from typical probe based systems which only use a short oligonucleotide sequence to target nucleic acid.

Within the DNA array (cDNA forest) there is an optimum distance between the DNA molecules so that they do not interact with each other. This distance is influenced by the length of the cDNA expected so that it is optimal that any two points need to be about twice the length of the longest cDNA from each other. For example, when the biological sample is (human) blood, the maximum length of RNA is around 5 kb so the maximum length of the cDNA produced therefrom will be around 5 kb. Thus, at least 10 kb (6000 nm) would be the optimal spacing between the oligonucleotides in the oligonucleotide array and/or cDNA molecules in the DNA array. Where oligonucleotides complementary to a target DNA or RNA are used, the distance between the oligonucleotides can be smaller as the known sequences allow for designing of the oligonucleotide sequences so there is minimal interaction. Accordingly where oligonucleotides complementary to a target DNA or RNA are used, the distance between the oligonucleotides may be at least 1.1, at least 1.2, at least 1.3, at least 1.4, at least 1.5, at least 1.6, at least 1.7, at least 1.8 or at least 1.9 times the length of the oligonucleotides.

As noted above the density of oligonucleotides in the oligonucleotide array influences the density of DNA molecules in the DNA array. Thus, one means of preventing the DNA molecules in the DNA array from interacting with each other is using a certain spacing (i.e. a maximum density) of oligonucleotides in the array as discussed above. The density of DNA molecules in the DNA array is also influenced by the concentration of RNA or cDNA molecules in the first RNA or first cDNA sample respectively. This concentration influences how many oligonucleotides in the oligonucleotide array capture an RNA molecule or cDNA molecule, as appropriate. This in turn influences how many DNA molecules are synthesised using the captured RNA or DNA as a template. Thus, the concentration of RNA or cDNA molecules in the first RNA or first cDNA sample may be adjusted to prevent the DNA molecules in the DNA array from interacting with each other.

Performance is also based on the ratio between the RNA and the DNA array (cDNA forest). Thermal control and kinetic control are relevant to optimum performance. A micropump can be used to generate laminar flow or turbulent flow.

Methods of RNA extraction and processing as described above may be combined and incorporated into pipelines for analysing biological samples. The method comprises:
(a) extracting RNA from the biological sample;
(b) preparing a processed RNA sample; and
(c) sequencing the processed RNA.

Blood or another liquid biopsy sample is collected from the subject in a container with cell lysis buffer and RNA stabilizing reagents. RNA stabilizing reagents are commercially available and include RNAlater^{®} (Sigma-Aldrich) and RNAprotect (Qiagen). A suitable buffer contains EDTA, sodium citrate and ammonium sulfate. Incubation for cell lysis can be, for example, 1 minute to 3 hours. The sample is then added to an RNA processing device as described above.

First RNA extraction (purification) takes place. The first reaction chamber (also termed flowcell or module herein) purifies the solution for RNA using oligonucleotides that can either be oligo-dT or random sequences. These oligonucleotides are bound to the surface of the chamber to make an oligo-forest. After the sample solution is pumped into the first chamber the chamber is heated to somewhere between 30°C and 75°C (optionally between 30°C and 65°C or between 60°C and 65°C) to allow for annealing of RNA to the oligo-forest. After the incubation period the remaining fluid is flushed out to a waste channel. The chamber is then heated to above 75°C (for example, between 80°C and 100°C) to release the remaining annealed RNA. This is then pumped through to the second reaction chamber.

The next step is preparing a processed RNA sample, in this case RNA normalization. The second reaction chamber (flowcell, module) has another oligo-forest. The purified RNA is cooled down in this chamber to below 65°C (i.e. 65°C or below, for example between 30°C and 65°C), optionally below 60°C, to allow for annealing to the oligo-forest. Reverse transcriptase and buffer is then added to create a complementary DNA strand using the oligo-forest as primers. Once the reverse transcription is completed the chamber is heated to between 80°C and 100°C (optionally above 90°C) to disassociate the RNA from the cDNA-forest. The solution is then flushed to waste channel. Another sample of purified RNA is then pumped into the second chamber with the cDNA-forest. The chamber is heated to between 45°C and 75°C (optionally between 60°C and 75°C) to allow for full length annealing of RNA to cDNA.

After incubation the non-annealed RNA is pumped into the collection chamber for further processing (to be sequenced). The second chamber is then heated to between 80°C and 100°C (optionally above 90°C) to release the RNA. The disassociated RNA is flushed to the waste channel. This process is repeated until an adequate amount of normalized RNA is produced for sequencing.

The next stage is preparation for sequencing. The normalized RNA can then be pumped into additional reaction chambers (flowcells, modules) which will prepare the sequencing libraries. Depending on the sequencing technology this could involve the ligation of adapters, second strand synthesis and/or any other required modifications to allow for sequencing. The sequencing libraries will then be pumped into a sequencing chamber for sequencing.

The next stage is sequencing and data processing. During sequencing the raw data can be uploaded to cloud servers for data processing and archiving.

Combining RNA extraction and processing in this way provides a device that can be used for immediate processing of blood or other samples minimizing issues with RNA degradation.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all embodiments described herein are considered to be broadly applicable and combinable with any and all other consistent embodiments, as appropriate.

Various publications are cited herein, the disclosures of which are incorporated by reference in their entireties.

## Claims

1. A method for determining a set of RNA sequences associated with a disease or condition, the method comprising:
(a) providing a test RNA sample extracted from a blood sample obtained from a subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with a disease or condition; and
(d) identifying a set of RNA sequences in the RNA transcript.

2. A method for producing a database of RNA sequences associated with a disease or condition, the method comprising:
(a) providing two or more test RNA samples extracted from blood samples obtained from one or more subjects;
(b) processing the RNA samples, optionally comprising synthesizing cDNA using the RNA as a template;
(c) sequencing the processed RNA or cDNA, wherein the sequencing output is used to identify the presence of two or more RNA transcript sequences associated with a disease or condition;
(d) identifying a set of RNA sequences in each RNA transcript; and
(e) compiling the sets of RNA sequences to form a database.

3. A method for diagnosing a disease or condition, wherein the method comprises:
(a) providing a test RNA sample extracted from a blood sample obtained from a subject;
(b) processing the RNA sample, optionally comprising synthesizing cDNA using the RNA as a template;
(c) sequencing the processed RNA or cDNA, wherein the sequencing output is used to determine the presence or absence of a set of RNA sequences that are unique to an RNA transcript sequence that is only expressed in subjects with the disease or condition.

4. A method for producing an RNA vaccine for a subject with a disease, the method comprising:
(a) providing a test RNA sample extracted from a blood sample obtained from the subject;
(b) processing the RNA sample, optionally comprising synthesizing cDNA using the RNA as a template;
(c) sequencing the processed RNA or cDNA, wherein the sequencing output is used to identify the presence of an RNA transcript sequence associated with the disease; and
(d) using the identified RNA transcript sequence to produce a first RNA vaccine for the subject;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

5. The method of claim 4, wherein the RNA transcript encodes a protein isoform present in the subject with the disease.

6. The method of claim 4 or 5, wherein using the identified RNA transcript sequence to produce the RNA vaccine comprises producing an RNA molecule comprising at least a portion of the RNA transcript sequence, wherein the RNA molecule comprises an open reading frame (ORF) encoding at least one antigenic peptide.

7. An RNA vaccine for use in therapy, wherein the RNA vaccine is produced using the method of any of claims 4 to 6.

8. A method for discovering a disease biomarker, the method comprising:
(a) providing a test RNA sample extracted from a blood sample obtained from a subject with the disease;
(b) processing the RNA sample, optionally comprising synthesizing cDNA using the RNA as a template; and
(c) sequencing the processed RNA or cDNA, wherein the sequencing output is used to discover a disease biomarker;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

9. A method for diagnosing cancer in a subject, the method comprising:
(a) providing a test RNA sample extracted from a blood sample obtained from the subject;
(b) processing the RNA sample, optionally comprising synthesizing cDNA using the RNA as a template; and
(c) sequencing the processed RNA or cDNA, wherein the sequencing output is used to identify whether the subject has cancer;
wherein processing the RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

10. The method of claim 9, wherein the sequencing output is used to determine the presence or absence of one or more RNA molecules in the RNA sample in order to identify whether the subject has cancer.

11. Use of a method for processing a test RNA or cDNA sample in a method for determining soil microfauna composition, wherein the method for processing an RNA or cDNA sample comprises:
(i) contacting the RNA or cDNA sample with a DNA array, comprising two or more DNA molecules, wherein one or more RNA or cDNA molecules from the RNA or cDNA sample anneal to the DNA molecules of the DNA array; and
(ii) extracting the unannealed RNA or cDNA molecules thereby generating processed RNA or cDNA.

12. The method of any one of claims 4-10 or use of claim 11, wherein the DNA array was produced by a method comprising:
(i) contacting a preparatory RNA sample with an oligonucleotide array, wherein the oligonucleotide array comprises two or more oligonucleotides linked to a surface, and wherein two or more RNA molecules from the RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules; and
(iv) removing the RNA sample from the surface.

13. The method of claim 8 or 12 further comprising:
(a) providing a control RNA sample extracted from a blood sample obtained from a subject without the disease;
(b) processing the control RNA sample, optionally comprising synthesizing cDNA using the RNA as a template; and
(c) sequencing the processed RNA or cDNA derived from the control RNA sample and comparing the sequencing output for the test RNA sample and control RNA sample to discover the disease biomarker.

14. The method of claim 12 or 13 wherein the preparatory RNA sample and the test RNA sample are derived from the same subject, optionally wherein the preparatory RNA sample and the test RNA sample are derived from the same blood sample.

15. The method of any of claims 1 to 10 or 12 to 14 or use of claim 11 wherein the RNA is full-length RNA and/or wherein sequencing comprises the use of long read sequencing.
